# EUROPEAN PATENT APPLICATION

(11) **EP 1 820 863 A1**
(43) Date of publication of application: **22.08.2007**
(21) Application number: 07001271.1
(22) Date of filing: 02.08.2001
(51) Int. Cl.: C12P 17/04, C12N 1/19, C12N 15/53, C12N 15/55, C12R 1/645, C12R 1/865

(54) **Ascorbic acid production from yeasts**

(30) Priority: 02.08.2000 US 630983
(62) Divisional of application: 04023458.5
(71) Applicant: Tate & Lyle Ingredients Americas, Inc., Decatur, IL 62521 (US)
(72) Inventor: Porro, Danilo, 22036 Erba (CO) (IT); Sauer, Michael, 6841 Maeder (AT)
(74) Representative: Fairbairn, Angus Chisholm

(57) **Abstract**

Herein is disclosed a method of generating ascorbic acid from yeast. In one embodiment, the yeast is a Saccharomyces or a Zygosaccharomyces spp. cultured in a medium comprising an ascorbic acid precursor. In a second embodiment the yeast is a recombinant yeast growing in a medium comprising an ascorbic acid precursor. The recombinant yeast is transformed with a coding region encoding an enzyme D-arabinose dehydrogenase (ARA), and may be further transformed with a coding region encoding an enzyme 1-galactono-1,4-lactone dehydrogenase (AGD) or D-arabinono-1,4-lactone oxidase (ALO). The ascorbic acid precursor is preferably D-glucose, L-galactose, L-galactono-1,4-lactone, L-gulono-1,4-lactone, or L-gulonic acid. In another preferred embodiment the ascorbic acid is accumulated in the medium at levels greater than background. Preferably, the yeast is capable of converting about 25 % of the ascorbic acid precursor to L-ascorbic acid.

SEQUENCE LISTING

<110> BIOPOLO S.C.a.R.L.

<120> Ascorbic Acid Production from Yeasts

<130> WPP84333

<140>
<141>

<150> US 09/630,983
<151> 2000-08-02

<160> 30

<170> PatentIn Ver. 2.1

<210> 1
<211> 610
<212> PRT
<213> Arabidopsis thaliana

<400> 1
Met Leu Arg Ser Leu Leu Leu Arg Arg Ser Val Gly His Ser Leu Gly
  1               5                  10                  15

Thr Leu Ser Pro Ser Ser Ser Thr Ile Arg Ser Ser Phe Ser Pro His
             20                  25                  30

Arg Thr Leu Cys Thr Thr Gly Gln Thr Leu Thr Pro Pro Pro Pro Pro
         35                  40                  45

Pro Pro Arg Pro Pro Pro Pro Pro Pro Ala Thr Ala Ser Glu Ala Gln
     50                  55                  60

Phe Arg Lys Tyr Ala Gly Tyr Ala Ala Leu Ala Ile Phe Ser Gly Val
 65                  70                  75                  80

Ala Thr Tyr Phe Ser Phe Pro Phe Pro Glu Asn Ala Lys His Lys Lys
                 85                  90                  95

Ala Gln Ile Phe Arg Tyr Ala Pro Leu Pro Glu Asp Leu His Thr Val
            100                 105                 110

Ser Asn Trp Ser Gly Thr His Glu Val Gln Thr Arg Asn Phe Asn Gln
        115                 120                 125

Pro Glu Asn Leu Ala Asp Leu Glu Ala Leu Val Lys Glu Ser His Glu
    130                 135                 140

Lys Lys Leu Arg Ile Arg Pro Val Gly Ser Gly Leu Ser Pro Asn Gly
145                 150                 155                 160

Ile Gly Leu Ser Arg Ser Gly Met Val Asn Leu Ala Leu Met Asp Lys
                165                 170                 175

Val Leu Glu Val Asp Lys Glu Lys Lys Arg Val Thr Val Gln Ala Gly
            180                 185                 190

Ile Arg Val Gln Gln Leu Val Asp Ala Ile Lys Asp Tyr Gly Leu Thr
        195                 200                 205

Leu Gln Asn Phe Ala Ser Ile Arg Glu Gln Gln Ile Gly Gly Ile Ile
    210                 215                 220

Gln Val Gly Ala His Gly Thr Gly Ala Arg Leu Pro Pro Ile Asp Glu
225                 230                 235                 240

Gln Val Ile Ser Met Lys Leu Val Thr Pro Ala Lys Gly Thr Ile Glu
                245                 250                 255

Leu Ser Arg Glu Lys Asp Pro Glu Leu Phe His Leu Ala Arg Cys Gly
            260                 265                 270

Leu Gly Gly Leu Gly Val Val Ala Glu Val Thr Leu Gln Cys Val Ala
        275                 280                 285

Arg His Glu Leu Val Glu His Thr Tyr Val Ser Asn Leu Gln Glu Ile
    290                 295                 300

Lys Lys Asn His Lys Lys Leu Leu Ser Ala Asn Lys His Val Lys Tyr
305                 310                 315                 320

Leu Tyr Ile Pro Tyr Thr Asp Thr Val Val Val Val Thr Cys Asn Pro
                325                 330                 335

Val Ser Lys Trp Ser Gly Pro Pro Lys Asp Lys Pro Lys Tyr Thr Thr
            340                 345                 350

Asp Glu Ala Val Gln His Val Arg Asp Leu Tyr Arg Glu Ser Ile Val
        355                 360                 365

Lys Tyr Arg Val Gln Asp Ser Gly Lys Lys Ser Pro Asp Ser Ser Glu
    370                 375                 380

Pro Asp Ile Gln Glu Leu Ser Phe Thr Glu Leu Arg Asp Lys Leu Leu
385                 390                 395                 400

Ala Leu Asp Pro Leu Asn Asp Val His Val Ala Lys Val Asn Gln Ala
                405                 410                 415

Glu Ala Glu Phe Trp Lys Lys Ser Glu Gly Tyr Arg Val Gly Trp Ser
            420                 425                 430

Asp Glu Ile Leu Gly Phe Asp Cys Gly Gly Gln Gln Trp Val Ser Glu
        435                 440                 445

Ser Cys Phe Pro Ala Gly Thr Leu Ala Asn Pro Ser Met Lys Asp Leu
    450                 455                 460

Glu Tyr Ile Glu Glu Leu Lys Lys Leu Ile Glu Lys Glu Ala Ile Pro
465                 470                 475                 480

Ala Pro Ala Pro Ile Glu Gln Arg Trp Thr Ala Arg Ser Lys Ser Pro
                485                 490                 495

Ile Ser Pro Ala Phe Ser Thr Ser Glu Asp Asp Ile Phe Ser Trp Val
            500                 505                 510

Gly Ile Ile Met Tyr Leu Pro Thr Ala Asp Pro Arg Gln Arg Lys Asp
        515                 520                 525

Ile Thr Asp Glu Phe Phe His Tyr Arg His Leu Thr Gln Lys Gln Leu
    530                 535                 540

Trp Asp Gln Phe Ser Ala Tyr Glu His Trp Ala Lys Ile Glu Ile Pro
545                 550                 555                 560

Lys Asp Lys Glu Glu Leu Glu Ala Leu Gln Ala Arg Ile Arg Lys Arg
                565                 570                 575

Phe Pro Val Asp Ala Tyr Asn Lys Ala Arg Arg Glu Leu Asp Pro Asn
            580                 585                 590

Arg Ile Leu Ser Asn Asn Met Val Glu Lys Leu Phe Pro Val Ser Thr
        595                 600                 605

Thr Ala
    610


<210> 2
<211> 1833
<212> DNA
<213> Artificial Sequence

<220>
<223> Description of Artificial Sequence: A. thaliana

<400> 2
atgctccggt cacttcttct ccgacgctcc gtcggccatt ctctcggaac cctatctccg 60
tcttcatcca ccatccgttc ctcattttcg cctcatcgta ctctctgcac caccggtcaa 120
acattgacac caccaccgcc gccaccgcca cgtcctccac ctccgcctcc ggccaccgcc 180
tcagaagctc aattccgtaa atacgccgga tacgcagcac tcgctatctt ctctggagtt 240
gctacctatt tctcatttcc attccctgag aatgctaaac acaagaaggc tcaaatcttc 300
cgttacgctc ctttacctga agatcttcac actgtctcta attggagtgg tactcatgag 360
gtacagacta ggaactttaa tcaaccggag aatcttgctg atctcgaagc tcttgttaag 420
gaatctcatg agaagaagtt aaggattcgt cccgttggat cgggtctctc gcctaatggg 480
attggtttgt ctcgctctgg gatggtgaat ctggcgctta tggataaagt tctagaggtg 540
gataaagaga agaagagagt tacggtgcag gctgggatta gggtccagca attggttgac 600
gccattaaag actatggtct tactcttcag aactttgcct ccattagaga gcagcagatt 660

ggtggtatta ttcaggttgg ggcacatggg acaggtgcta gattgcctcc tattgatgag 720
caggtgatca gtatgaagct ggttactcct gcgaagggaa caattgaact ttcaagagag 780
aaagatccgg agctctttca tctagctcga tgtggccttg gtggacttgg agttgttgct 840
gaggtcaccc tccaatgcgt tgcaagacat gaacttgtgg aacacacata cgtctcaaac 900
ttgcaagaaa tcaagaaaaa tcacaaaaaa ttgctctctg caaacaagca tgttaagtac 960
ctatatattc cttataccga cacagtcgtg gttgtaacat gcaatcctgt atcaaaatgg 1020
agtgggccac ctaaggacaa accaaagtac actacagatg aggctgtaca gcatgtccgt 1080
gatctctaca gagagagcat tgtgaagtat agggtccagg actctggtaa gaagtctcct 1140
gacagcagtg agccagacat acaggagctt tcatttacag agttgagaga caaactactt 1200
gcccttgatc ctctcaatga cgttcacgtt gcaaaagtaa atcaagctga ggcagagttt 1260
tggaaaaaat cagaaggata tagagtaggg tggagtgatg aaattctggg ctttgactgt 1320
ggtggtcagc agtgggtgtc agaatcttgt tttcctgctg gaaccctcgc caaccctagc 1380
atgaaagacc ttgaatacat agaagagctg aaaaaactaa tagaaaagga agcaatacca 1440
gcacctgctc caatagagca gcgatggaca gctcgaagta agagccccat tagtcctgca 1500
ttcagcactt cagaggatga tattttctca tgggttggta taatcatgta cctcccgaca 1560
gcagaccctc gccagagaaa ggacatcaca gatgaatttt tccactatag acatttgaca 1620
cagaaacaat tgtgggatca attttctgcg tatgaacatt gggctaaaat tgagatacca 1680
aaagacaaag aagaacttga agccttacag gcaagaataa gaaaacgttt cccagtggat 1740
gcatacaaca aagcacgtag ggagctggac ccaaatagaa tcctctccaa caacatggtg 1800
gaaaagctct tcccagtctc caccactgct taa 1833

<210> 3
<211> 600
<212> PRT
<213> Brassica oleracea

<400> 3
Met Leu Arg Ser Leu Leu Leu Arg Arg Ser Asn Ala Arg Ser Leu Arg
1               5                   10                  15

Pro Pro Phe Pro Pro Leu Arg Thr Leu Cys Thr Ser Gly Gln Thr Leu
            20                  25                  30

Thr Pro Ala Pro Pro Pro Pro Pro Pro Pro Pro Pro Pro Ile Ser Ser
        35                  40                  45

Ser Ala Ser Glu Lys Glu Phe Arg Lys Tyr Ala Gly Tyr Ala Ala Leu
    50                  55                  60

Ala Leu Phe Ser Gly Ala Ala Thr Tyr Phe Ser Phe Pro Phe Pro Glu
65                  70                  75                  80

Asn Ala Lys His Lys Lys Ala Gln Ile Phe Arg Tyr Ala Pro Leu Pro
                85                  90                  95

Glu Asp Leu His Thr Val Ser Asn Trp Ser Gly Thr His Glu Val Gln
            100                 105                 110

Thr Arg Asn Phe Asn Gln Pro Glu Thr Leu Ala Asp Leu Glu Ala Leu
        115                 120                 125

Val Lys Glu Ala His Glu Lys Lys Asn Arg Ile Arg Pro Val Gly Ser
    130                 135                 140

Gly Leu Ser Pro Asn Gly Ile Gly Leu Ser Arg Ser Gly Met Val Asn
145                 150                 155                 160

Leu Ala Leu Met Asp Lys Val Leu Glu Val Asp Lys Glu Lys Lys Arg
                165                 170                 175

Val Arg Val Gln Ala Gly Ile Arg Val Gln Gln Leu Val Asp Ala Ile
            180                 185                 190

Gln Glu Tyr Gly Leu Thr Leu Gln Asn Phe Ala Ser Ile Arg Glu Gln
        195                 200                 205

Gln Ile Gly Gly Ile Ile Gln Val Gly Ala His Gly Thr Gly Ala Arg
    210                 215                 220

Leu Pro Pro Ile Asp Glu Gln Val Ile Gly Met Lys Leu Val Thr Pro
225                 230                 235                 240

Ala Lys Gly Thr Ile Glu Leu Ser Lys Asp Asn Asp Pro Glu Leu Phe
                245                 250                 255

His Leu Ala Arg Cys Gly Leu Gly Gly Leu Gly Val Val Ala Glu Val
            260                 265                 270

Thr Leu Gln Cys Val Glu Arg Gln Glu Leu Leu Glu His Thr Tyr Val
        275                 280                 285

Ser Thr Leu Glu Glu Ile Lys Lys Asn His Lys Lys Leu Leu Ser Thr
    290                 295                 300

Asn Lys His Val Lys Tyr Leu Tyr Ile Pro Tyr Thr Asp Thr Val Val
305                 310                 315                 320

Val Val Thr Cys Asn Pro Val Ser Lys Trp Ser Gly Ala Pro Lys Asp
                325                 330                 335

Lys Pro Lys Tyr Thr Thr Glu Glu Ala Leu Lys His Val Arg Asp Leu
            340                 345                 350

Tyr Arg Glu Ser Ile Val Lys Tyr Arg Val Gln Asp Ser Ser Lys Lys
        355                 360                 365

Thr Pro Asp Ser Arg Glu Pro Asp Ile Asn Glu Leu Ser Phe Thr Glu
    370                 375                 380

Leu Arg Asp Lys Leu Ile Ala Leu Asp Pro Leu Asn Asp Val His Val
385                 390                 395                 400

Gly Lys Val Asn Gln Ala Glu Ala Glu Phe Trp Lys Lys Ser Glu Gly
                405                 410                 415

Tyr Arg Val Gly Trp Ser Asp Glu Ile Leu Gly Phe Asp Cys Gly Gly
            420                 425                 430

Gln Gln Trp Val Ser Glu Thr Cys Phe Pro Ala Gly Thr Leu Ala Lys
        435                 440                 445

Pro Ser Met Lys Asp Leu Glu Tyr Ile Glu Gln Leu Lys Glu Leu Ile
    450                 455                 460

Gln Lys Glu Ala Ile Pro Ala Pro Ser Pro Ile Glu Gln Arg Trp Thr
465                 470                 475                 480

Gly Arg Ser Lys Ser Pro Met Ser Pro Ala Phe Ser Thr Ala Glu Glu
                485                 490                 495

Asp Ile Phe Ser Trp Val Gly Ile Ile Met Tyr Leu Pro Thr Ala Asp
            500                 505                 510

Pro Arg Gln Arg Lys Asp Ile Thr Asp Glu Phe Phe His Tyr Arg His
        515                 520                 525

Leu Thr Gln Ala Lys Leu Trp Asp Gln Tyr Ser Ala Tyr Glu His Trp
    530                 535                 540

Ala Lys Ile Glu Ile Pro Lys Asp Lys Glu Glu Leu Glu Ala Leu Gln
545                 550                 555                 560

Glu Arg Leu Arg Lys Arg Phe Pro Val Asp Ala Tyr Asn Lys Ala Arg
                565                 570                 575

Arg Glu Leu Asp Pro Asn Arg Ile Leu Ser Asn Asn Met Val Glu Lys
            580                 585                 590

Leu Phe Pro Val Ser Lys Thr Ala
        595                 600

<210> 4
<211> 2064
<212> DNA
<213> Brassica oleracea

<400> 4
aattcggcac gagctttcgc tggctcaggt ttcagatcgc ctgaactaaa acaaaatgct 60
ccgatcactt ctcctccgcc gctccaacgc ccgttcgctt cgacccccat ttccccctct 120
ccgcactcta tgcacttccg gtcagacctt gactccagcc cctccaccgc cgcctcctcc 180
tccaccgccg atttcatcct ccgcctcaga aaaggagttc cgtaaatacg ccggatacgc 240
agcactcgct ctcttctccg gcgccgcaac ttacttctcc ttccccttcc ccgagaacgc 300
caaacacaag aaggctcaga tcttccgata cgctcctctc cccgaagatc tccacaccgt 360
ctctaactgg agtggtactc acgaggtcca gaccaggaac tttaaccagc cggagactct 420
cgccgatctc gaagctctcg tcaaggaagc tcatgagaag aagaacagga tccgacccgt 480
tggatccggt ctttccccca atgggatcgg tttgtctcgc tcggggatgg tgaatttggc 540
gctcatggac aaggtcctcg aggtggataa agagaagaag agagtccgtg tgcaggctgg 600
gattagggtt cagcagcttg ttgacgccat tcaagagtat ggtctcactc tccagaactt 660
tgcttccatt agagagcagc agattggtgg catcattcag gttggggcac atgggacagg 720
tgctagattg cctcctatcg atgagcaagt gattggcatg aagcttgtca ctcctgctaa 780

gggaactatt gagctttcta aggataatga tccggagctc tttcatcttg ctcgatgtgg 840
ccttggtgga cttggagttg ttgctgaggt caccctccag tgcgttgaaa gacaggagct 900
tttggagcac acttacgtct ccaccttgga agagatcaag aaaaatcaca aaaagttgct 960
ctctacaaat aagcatgtca agtacctgta tattccatat actgacacgg tcgtggttgt 1020
tacatgcaac cctgtatcaa aatggagtgg ggcacctaag gacaaaccaa agtacactac 1080
agaggaggct ttaaagcatg tccgtgacct gtatagagag agcattgtta agtatagggt 1140
ccaggactct agtaagaaga ctcctgacag tagggagcca gacattaacg agctttcatt 1200
tacagagttg agagataagc tgattgccct agatcctctc aatgacgttc acgttggaaa 1260
agtgaatcaa gctgaggctg agttttggaa aaaatcagaa ggatacagag tagggtggag 1320
tgatgaaatc ctgggctttg actgtggtgg tcaacagtgg gtatcagaaa cttgttttcc 1380
tgctggaact ctcgctaaac ctagcatgaa agaccttgag tacatagaac agctgaaaga 1440
gttgatacaa aaagaagcaa taccagcacc ttctcccata gagcagcgtt ggacaggccg 1500
aagtaagagc cctatgagtc ctgcattcag cactgcagag gaggacattt tctcatgggt 1560
tggtataatc atgtatctcc cgacagcaga ccctcgccag agaaaggaca tcacggatga 1620
atttttccac tatagacatt tgacacaggc aaaattgtgg gaccagtatt ctgcgtatga 1680
acattgggct aaaattgaga taccaaagga taaagaggaa cttgaagccc tacaagaaag 1740
actcagaaaa cgattcccgg tggatgcata caacaaagca cgaagggagc tggacccaaa 1800
cagaattctc tcaaacaaca tggtggaaaa gctcttccct gtctccaaga ctgcttaaaa 1860
acattttcat caatagtttt tttgctcctt gaagtaccac ttttggaatc ctataacgtt 1920
gcatctacaa gtgtttgtaa gaagagtgaa gccgatatat tggtcacaaa aaaagtttac 1980
attgagtttt actactattt tttttttcgc agttcccctg aataaatata cttgttgttc 2040
tattccaaaa aaaaaaaaaa aaaa 2064

<210> 5
<211> 526
<212> PRT
<213> Saccharomyces cerevisiae

<400> 5
Met Ser Thr Ile Pro Phe Arg Lys Asn Tyr Val Phe Lys Asn Trp Ala
1               5                   10                  15

Gly Ile Tyr Ser Ala Lys Pro Glu Arg Tyr Phe Gln Pro Ser Ser Ile
            20                  25                  30

Asp Glu Val Val Glu Leu Val Lys Ser Ala Arg Leu Ala Glu Lys Ser
        35                  40                  45

Leu Val Thr Val Gly Ser Gly His Ser Pro Ser Asn Met Cys Val Thr
    50                  55                  60

Asp Glu Trp Leu Val Asn Leu Asp Arg Leu Asp Lys Val Gln Lys Phe
65                  70                  75                  80

Val Glu Tyr Pro Glu Leu His Tyr Ala Asp Val Thr Val Asp Ala Gly
                85                  90                  95

Met Arg Leu Tyr Gln Leu Asn Glu Phe Leu Gly Ala Lys Gly Tyr Ser
            100                 105                 110

Ile Gln Asn Leu Gly Ser Ile Ser Glu Gln Ser Val Ala Gly Ile Ile
        115                 120                 125

Ser Thr Gly Ser His Gly Ser Ser Pro Tyr His Gly Leu Ile Ser Ser
    130                 135                 140

Gln Tyr Val Asn Leu Thr Ile Val Asn Gly Lys Gly Glu Leu Lys Phe
145                 150                 155                 160

Leu Asp Ala Glu Asn Asp Pro Glu Val Phe Lys Ala Ala Leu Leu Ser
                165                 170                 175

Val Gly Lys Ile Gly Ile Ile Val Ser Ala Thr Ile Arg Val Val Pro
            180                 185                 190

Gly Phe Asn Ile Lys Ser Thr Gln Glu Val Ile Thr Phe Glu Asn Leu
        195                 200                 205

Leu Lys Gln Trp Asp Thr Leu Trp Thr Ser Ser Glu Phe Ile Arg Val
    210                 215                 220

Trp Trp Tyr Pro Tyr Thr Arg Lys Cys Val Leu Trp Arg Gly Asn Lys
225                 230                 235                 240

Thr Thr Asp Ala Gln Asn Gly Pro Ala Lys Ser Trp Trp Gly Thr Lys
                245                 250                 255

Leu Gly Arg Phe Phe Tyr Glu Thr Leu Leu Trp Ile Ser Thr Lys Ile
            260                 265                 270

Tyr Ala Pro Leu Thr Pro Phe Val Glu Lys Phe Val Phe Asn Arg Gln
        275                 280                 285

Tyr Gly Lys Leu Glu Lys Ser Ser Thr Gly Asp Val Asn Val Thr Asp
    290                 295                 300

Ser Ile Ser Gly Phe Asn Met Asp Cys Leu Phe Ser Gln Phe Val Asp
305                 310                 315                 320

Glu Trp Gly Cys Pro Met Asp Asn Gly Leu Glu Val Leu Arg Ser Leu
                325                 330                 335

Asp His Ser Ile Ala Gln Ala Ala Ile Asn Lys Glu Phe Tyr Val His
            340                 345                 350

Val Pro Met Glu Val Arg Cys Ser Asn Thr Thr Leu Pro Ser Glu Pro
        355                 360                 365

Leu Asp Thr Ser Lys Arg Thr Asn Thr Ser Pro Gly Pro Val Tyr Gly
    370                 375                 380

Asn Val Cys Arg Pro Phe Leu Asp Asn Thr Pro Ser His Cys Arg Phe
385                 390                 395                 400

Ala Pro Leu Glu Asn Val Thr Asn Ser Gln Leu Thr Leu Tyr Ile Asn
                405                 410                 415

Ala Thr Ile Tyr Arg Pro Phe Gly Cys Asn Thr Pro Ile His Lys Trp
            420                 425                 430

Phe Thr Leu Phe Glu Asn Thr Met Met Val Ala Gly Gly Lys Pro His
        435                 440                 445

Trp Ala Lys Asn Phe Leu Gly Ser Thr Thr Leu Ala Ala Gly Pro Val
    450                 455                 460

Lys Lys Asp Thr Asp Tyr Asp Asp Phe Glu Met Arg Gly Met Ala Leu
465                 470                 475                 480

Lys Val Glu Glu Trp Tyr Gly Glu Asp Leu Lys Lys Phe Arg Lys Ile
                485                 490                 495

Arg Lys Glu Gln Asp Pro Asp Asn Val Phe Leu Ala Asn Lys Gln Trp
            500                 505                 510

Ala Ile Ile Asn Gly Ile Ile Asp Pro Ser Glu Leu Ser Asp
        515                 520                 525

<210> 6
<211> 1581
<212> DNA
<213> Saccharomyces cerevisiae

<400> 6
atgtctacta tcccatttag aaagaactat gtgttcaaaa actgggccgg aatttattct 60
gcaaaaccag aacgttactt ccaaccaagt tcaattgatg aggttgtcga gttagtaaag 120
agtgccaggc tagctgaaaa aagcttagtt actgttggtt cgggccattc tcctagtaac 180
atgtgcgtta ctgatgaatg gcttgttaac ttagacagat tggacaaagt acaaaagttt 240
gttgaatatc ctgagttaca ttatgccgat gtcacagttg atgccggtat gaggctttac 300
caattgaatg aatttttggg tgcgaaaggt tactctatcc aaaatttagg ctctatctca 360
gaacaaagtg ttgctggcat aatctctact ggtagtcatg gttcctcacc ttatcacggt 420
ttgatttctt ctcaatacgt aaacttgact attgttaatg gtaagggcga attgaagttc 480
ttggatgccg aaaacgatcc agaagtcttt aaagctgctt tactttcagt tggaaaattt 540
ggtatcattg tctctgctac tatcagggtt gttcccggct tcaatattaa atccactcaa 600
gaagtgatta cttttgaaaa ccttttgaag caatgggata ccctatggac ttcatctgaa 660
tttatcagag tttggtggta cccttatact agaaaatgtg ttctatggag gggtaacaaa 720
actacagatg cccaaaatgg tccagccaag tcatggtggg gtaccaagct gggtagattt 780
ttctacgaaa ctctattatg gatctctacc aaaatctatg cgccattaac cccatttgtg 840
gaaaagttcg ttttcaacag gcaatatggg aaattggaga agagctctac tggtgatgtt 900
aatgttaccg attctatcag cggatttaat atggactgtt tgttttcaca atttgttgat 960
gaatgggggt gccctatgga taatggtttg gaagtcttac gttcattgga tcattctatt 1020
gcgcaggctg ccataaacaa agaattttat gtccacgtgc ctatggaagt ccgttgctca 1080
aatactacat taccttctga acccttggat actagcaaga gaacaaacac cagtcccggt 1140
cccgtttatg gcaatgtgtg ccgcccattc ctggataaca caccatccca ttgcagattt 1200
gctccgttgg aaaatgttac caacagtcag ttgacgttgt acataaatgc taccatttat 1260
aggccgtttg gctgtaatac tccaattcat aaatggttta ccctttttga aaatactatg 1320
atggtagcgg gaggtaagcc acattgggcc aagaacttcc taggctcaac cactctagct 1380
gctggaccag tgaaaaagga tactgattac gatgactttg aaatgagggg gatggcattg 1440
aaggttgaag aatggtatgg cgaggatttg aaaaagttcc ggaaaataag aaaggagcaa 1500
gatcccgata atgtattctt ggcaaacaaa cagtgggcta tcataaatgg tattatagat 1560

cctagtgagt tgtccgacta g                                                1581

<210> 7
<211> 526
<212> PRT
<213> Saccharomyces cerevisiae

<400> 7
Met Ser Thr Ile Pro Phe Arg Lys Asn Tyr Val Phe Lys Asn Trp Ala
1               5                   10                  15

Gly Ile Tyr Ser Ala Lys Pro Glu Arg Tyr Phe Gln Pro Ser Ser Ile
            20                  25                  30

Asp Glu Val Val Glu Leu Val Lys Ser Ala Arg Leu Ala Glu Lys Ser
        35                  40                  45

Leu Val Thr Val Gly Ser Gly His Ser Pro Ser Asn Met Cys Val Thr
    50                  55                  60

Asp Glu Trp Leu Val Asn Leu Asp Arg Leu Asp Lys Val Gln Lys Phe
65                  70                  75                  80

Val Glu Tyr Pro Glu Leu His Tyr Ala Asp Val Thr Val Asp Ala Gly
                85                  90                  95

Met Arg Leu Tyr Gln Leu Asn Glu Phe Leu Gly Ala Lys Gly Tyr Ser
            100                 105                 110

Ile Gln Asn Leu Gly Ser Ile Ser Glu Gln Ser Val Ala Gly Ile Ile
        115                 120                 125

Ser Thr Gly Ser His Gly Ser Ser Pro Tyr His Gly Leu Ile Ser Ser
    130                 135                 140

Gln Tyr Val Asn Leu Thr Ile Val Asn Gly Lys Gly Glu Leu Lys Phe
145                 150                 155                 160

Leu Asp Ala Glu Asn Asp Pro Glu Val Phe Lys Ala Ala Leu Leu Ser
                165                 170                 175

Val Gly Lys Ile Gly Ile Ile Val Ser Ala Thr Ile Arg Val Val Pro
            180                 185                 190

Gly Phe Asn Ile Lys Ser Thr Gln Glu Val Ile Thr Phe Glu Asn Leu
        195                 200                 205

Leu Lys Gln Trp Asp Thr Leu Trp Thr Ser Ser Glu Phe Ile Arg Val
    210                 215                 220

Trp Trp Tyr Pro Tyr Thr Arg Lys Cys Val Leu Trp Arg Gly Asn Lys
225                 230                 235                 240

Thr Thr Asp Ala Gln Asn Gly Pro Ala Lys Ser Trp Trp Gly Thr Lys
                245                 250                 255

Leu Gly Arg Phe Phe Tyr Glu Thr Leu Leu Trp Ile Ser Thr Lys Ile
            260                 265                 270

Tyr Ala Pro Leu Thr Pro Phe Val Glu Lys Phe Val Phe Asn Arg Gln
        275                 280                 285

Tyr Gly Lys Leu Glu Lys Ser Ser Thr Gly Asp Val Asn Val Thr Asp
    290                 295                 300

Ser Ile Ser Gly Phe Asn Met Asp Cys Leu Phe Ser Gln Phe Val Asp
305                 310                 315                 320

Glu Trp Gly Cys Pro Met Asp Asn Gly Leu Glu Val Leu Arg Ser Leu
                325                 330                 335

Asp His Ser Ile Ala Gln Ala Ala Ile Asn Lys Glu Phe Tyr Val His
            340                 345                 350

Val Pro Met Glu Val Arg Cys Ser Asn Thr Thr Leu Pro Ser Glu Pro
        355                 360                 365

Leu Asp Thr Ser Lys Arg Thr Asn Thr Ser Pro Gly Pro Val Tyr Gly
    370                 375                 380

Asn Val Cys Arg Pro Phe Leu Asp Asn Thr Pro Ser His Cys Arg Phe
385                 390                 395                 400

Ala Pro Leu Glu Asn Val Thr Asn Ser Gln Leu Thr Leu Tyr Ile Asn
                405                 410                 415

Pro Thr Ile Tyr Arg Pro Phe Gly Cys Asn Thr Pro Ile His Lys Trp
            420                 425                 430

Phe Thr Leu Phe Glu Asn Thr Met Met Val Ala Gly Gly Lys Pro His
        435                 440                 445

Trp Ala Lys Asn Phe Leu Gly Ser Thr Thr Leu Ala Ala Gly Pro Val
    450                 455                 460

Lys Lys Asp Thr Asp Tyr Asp Asp Phe Glu Met Arg Gly Met Ala Leu
465                 470                 475                 480

Lys Val Glu Glu Trp Tyr Gly Glu Asp Leu Lys Lys Phe Arg Lys Ile
                485                 490                 495

Arg Lys Glu Gln Asp Pro Asp Asn Val Phe Leu Ala Asn Lys Gln Trp
            500                 505                 510

Ala Ile Ile Asn Gly Ile Ile Asp Pro Ser Glu Leu Ser Asp
        515                 520                 525

<210> 8
<211> 2138
<212> DNA
<213> Saccharomyces cerevisiae

<400> 8
cccatgtcta ctatcccatt tagaaagaac tatgtgttca aaaactgggc cggaatttat 60
tctgcaaaac cagaacgtta cttccaacca agttcaattg atgaggttgt cgagttagta 120
aagagtgcca ggctagctga aaaaagctta gttactgttg gttcgggcca ttctcctagt 180
aacatgtgcg ttactgatga atggcttgtt aacttagaca gattggacaa agtacaaaag 240
tttgttgaat atcctgagtt acattatgcc gatgtcacag ttgatgccgg tatgaggctt 300
taccaattga atgaattttt gggtgcgaaa ggttactcta tccaaaattt aggctctatc 360
tcagaacaaa gtgttgctgg cataatctct actggtagtc atggttcctc accttatcac 420
ggtttgattt cttctcaata cgtaaacttg actattgtta atggtaaggg cgaattgaag 480
ttcttggatg ccgaaaacga tccagaagtc tttaaagctg ctttactttc agttggaaaa 540
atcggtatca ttgtctctgc tactatcagg gttgttcccg gcttcaatat taaatccact 600
caagaagtga ttacttttga aaaccttttg aagcaatggg ataccctatg gacttcatct 660
gaatttatca gagtttggtg gtacccttat actagaaaat gtgttctatg gaggggtaac 720
aaaactacag atgcccaaaa tggtccagcc aagtcatggt ggggtaccaa gctgggtaga 780
tttttctacg aaactctatt atggatctct accaaaatct atgcgccatt aaccccattt 840
gtggaaaagt tcgttttcaa caggcaatac gggaaattgg agaagagctc tactggtgat 900
gttaatgtta ccgattctat cagcggattt aatatggact gtttgttttc acaatttgtt 960
gatgaatggg ggtgccctat ggataatggt ttggaagtct tacgttcatt ggatcattct 1020
attgcgcagg ctgccataaa caaagaattt tatgtccacg tgcctatgga agtccgttgc 1080
tcaaatacta cattaccttc tgaacccttg gatactagca agagaacaaa caccagtccc 1140
ggtcccgttt atggcaatgt gtgccgccca ttcctggata acacaccatc ccattgcaga 1200
tttgctccgt tggaaaatgt taccaacagt cagttgacgt tgtacataaa tcctaccatt 1260
tataggccgt ttggctgtaa tactccaatt cataaatggt ttaccctttt tgaaaatact 1320
atgatggtag cgggaggtaa gccacattgg gccaagaact tcctaggctc aaccactcta 1380
gctgctggac cagtgaaaaa ggatactgat tacgatgact ttgaaatgag ggggatggca 1440
ttgaaggttg aagaatggta tggcgaggat ttgaaaaagt tccggaaaat aagaaaggag 1500
caagatcccg ataatgtatt cttggcaaac aaacagtggg ctatcataaa tggtattata 1560
gatcctagtg agttgtccga ctagtctctt tttgtctcaa taatctctat attttactaa 1620
aaaagaatat atatatatat atttatatat agcagtgtga tgactgttca tgtacattct 1680
aataactatt cctagctgcc tatcaaagac ttttttttga attagagctt tttagtaatc 1740
atgggaccct tttttctttt cattatcctt actatagttt ttttttggaa aagccgaacg 1800
cggtaatgat tggtcgtata agcaaaaacg aaacatcggc atggcataac gtagatccta 1860
tctacaggga agtttttaga aatcagatag aaatgtattt tgagtgctgt atatattgca 1920
gtactttttt tctctctagg atttaagtat gtttagtatt aactcatatc acattttttc 1980
tttgtaaaaa gcaaccattc gcaacaatgt cgatagtaga gacatgcata tcgtttgttt 2040
cgacaaatcc gttttatcca ttttgtactg gattgcttct gaattgtgtg gttacaccgc 2100
tttacttttg gaaaacgcaa aatggtagaa tcgtggtc 2138

<210> 9
<211> 440
<212> PRT
<213> Rattus norvegicus

<400> 9
Met Val His Gly Tyr Lys Gly Val Gln Phe Gln Asn Trp Ala Lys Thr
1               5                   10                  15

Tyr Gly Cys Ser Pro Glu Val Tyr Tyr Gln Pro Thr Ser Val Glu Glu
            20                  25                  30

Val Arg Glu Val Leu Ala Leu Ala Arg Glu Gln Lys Lys Lys Val Lys
        35                  40                  45

Val Val Gly Gly Gly His Ser Pro Ser Asp Ile Ala Cys Thr Asp Gly
    50                  55                  60

Phe Met Ile His Met Gly Lys Met Asn Arg Val Leu Gln Val Asp Lys
65                  70                  75                  80

Glu Lys Lys Gln Ile Thr Val Glu Ala Gly Ile Leu Leu Ala Asp Leu
                85                  90                  95

His Pro Gln Leu Asp Glu His Gly Leu Ala Met Ser Asn Leu Gly Ala
            100                 105                 110

Val Ser Asp Val Thr Val Ala Gly Val Ile Gly Ser Gly Thr His Asn
        115                 120                 125

Thr Gly Ile Lys His Gly Ile Leu Ala Thr Gln Val Val Ala Leu Thr
    130                 135                 140

Leu Met Thr Ala Asp Gly Glu Val Leu Glu Cys Ser Glu Ser Arg Asn
145                 150                 155                 160

Ala Asp Val Phe Gln Ala Ala Arg Val His Leu Gly Cys Leu Gly Ile
                165                 170                 175

Ile Leu Thr Val Thr Leu Gln Cys Val Pro Gln Phe Gln Leu Gln Glu
            180                 185                 190

Thr Ser Phe Pro Ser Thr Leu Lys Glu Val Leu Asp Asn Leu Asp Ser
        195                 200                 205

His Leu Lys Arg Ser Glu Tyr Phe Arg Phe Leu Trp Phe Pro His Thr
    210                 215                 220

Glu Asn Val Ser Ile Ile Tyr Gln Asp His Thr Asn Lys Ala Pro Ser
225                 230                 235                 240

Ser Ala Ser Asn Trp Phe Trp Asp Tyr Ala Ile Gly Phe Tyr Leu Leu
                245                 250                 255

Glu Phe Leu Leu Trp Thr Ser Thr Tyr Leu Pro Cys Leu Val Gly Trp
            260                 265                 270

Ile Asn Arg Phe Phe Phe Trp Met Leu Phe Asn Cys Lys Lys Glu Ser
        275                 280                 285

Ser Asn Leu Ser His Lys Ile Phe Thr Tyr Glu Cys Arg Phe Lys Gln
    290                 295                 300

His Val Gln Asp Trp Ala Ile Pro Arg Glu Lys Thr Lys Glu Ala Leu
305                 310                 315                 320

Leu Glu Leu Lys Ala Met Leu Glu Ala His Pro Lys Val Val Ala His
                325                 330                 335

Tyr Pro Val Glu Val Arg Phe Thr Arg Gly Asp Asp Ile Leu Leu Ser
            340                 345                 350

Pro Cys Phe Gln Arg Asp Ser Cys Tyr Met Asn Ile Ile Met Tyr Arg
        355                 360                 365

Pro Tyr Gly Lys Asp Val Pro Arg Leu Asp Tyr Trp Leu Ala Tyr Glu
    370                 375                 380

Thr Ile Met Lys Lys Phe Gly Gly Arg Pro His Trp Ala Lys Ala His
385                 390                 395                 400

Asn Cys Thr Gln Lys Asp Phe Glu Glu Met Tyr Pro Thr Phe His Lys
                405                 410                 415

Phe Cys Asp Ile Arg Glu Lys Leu Asp Pro Thr Gly Met Phe Leu Asn
            420                 425                 430

Ser Tyr Leu Glu Lys Val Phe Tyr
        435                 440

<210> 10
<211> 2120
<212> DNA
<213> Rattus norvegicus

<400> 10
   ggatcctcct gatcactgga atcatggtcc atgggtacaa aggggtccag ttccaaaatt 60
   gggcaaagac ctatggttgc agtccagagg tgtactacca gcccacctcc gtggaggagg 120
   tcagagaggt gctggccctg gcccgggagc agaagaagaa agtgaaggtg gtgggtggtg 180
   gccactcgcc ttcagacatt gcctgcactg acggtttcat gatccacatg ggcaagatga 240
   accgggttct ccaggtggac aaggagaaga agcagataac agtggaagcc ggtatcctcc 300
   tggctgacct gcacccacag ctggatgagc atggcctggc catgtccaat ctgggagcag 360
   tgtctgatgt gacagttgct ggtgtcattg gatccggaac acataacaca gggatcaagc 420
   acggcatcct ggccactcag gtggtggccc tgaccctgat gacagctgat ggagaagttc 480
   tggaatgttc tgagtcaaga aatgcagatg tgttccaggc tgcacgggtg cacctgggtt 540
   gcctgggcat catcctcacc gtcaccctgc agtgtgtgcc tcagtttcag cttcaggaga 600
   catccttccc ttcgaccctc aaagaggtcc ttgacaacct agacagccac ctgaagaggt 660
   ctgagtactt ccgcttcctc tggtttcctc acactgagaa cgtcagcatc atctaccaag 720
   accacaccaa caaggccccc tcctctgcat ctaactggtt ttgggactat gccatcgggt 780
   tctacctact ggagttcttg ctctggacca gcacctacct gccatgcctc gtgggctgga 840
   tcaaccgctt cttcttctgg atgctgttca actgcaagaa ggagagcagc aacctcagtc 900
   acaagatctt cacctacgag tgtcgcttca agcagcatgt acaagactgg gccatcccta 960
   gggagaagac caaggaggcc ctactggagc taaaggccat gctggaggcc caccccaaag 1020
   tggtagccca ctaccccgta gaggtgcgct tcacccgagg cgatgacatt ctgctgagcc 1080
   cctgcttcca gagggacagc tgctacatga acatcattat gtacaggccc tatggaaagg 1140
   acgtgcctcg gctagactac tggctggcct atgagaccat catgaagaag tttggaggaa 1200
   gaccccactg ggcaaaggcc cacaattgca cccagaagga ctttgaggaa atgtacccca 1260
   cctttcacaa gttctgtgac atccgtgaga agctggaccc cactggaatg ttcttgaatt 1320
   cgtacctgga gaaagtcttc tactaaagca ggagtggaaa caaaccaccc tgacccctca 1380
   cacttctgct gcccccgggg gtctggggag cagagaagtg cctcacaagc acaatgggaa 1440
   ctgacctctc ctcctgacca caaagaaagg ctgggctctg ggccgggtcc tctctgcctt 1500
   cggcatcatt tcccttacat ccaggcgaag aagtggcctc tcactcaaat tcctgttagc 1560
   atttccatgg gtcacacata aactgcaatc ctctcaggag aagggggatc cctgatacat 1620
   catatctatc cagactaagg atgtggttct tcctagattc tatggctcca ccaggtatag 1680
   agagattcct ggggcctgca gttctccatc cctcttcaga agggagggat cccttggcga 1740
   gagtttggct cagaggtggc atgaagcatg ctctgctctc tcttaccctt gaaggtcctt 1800
   cggatgccca gagatgtctg ctggtcctgg gcaagccatc attcaaacgg gtccaacctg 1860
   gccttctgtc tgccatggcc tgaccctcgc agtgtctctt ccagaggtgt ttagagtgga 1920
   actcgcttca acctcttaac cagttgctga tccctgtgtt tctctccctt ctccttggag 1980
   actactcttg gagggggatc ccaccatgtc cttggctttc cctgggtatt gttctcctct 2040
   tcctcttcac aaatatgatt tcagtttgat ttgtggcctt tctggagtgt tccttggaga 2100
   accaagatgt tccagctacc 2120

<210> 11
<211> 319
<212> PRT
<213> Arabidopsis thaliana

<400> 11
Met Thr Lys Ile Glu Leu Arg Ala Leu Gly Asn Thr Gly Leu Lys Val
1               5                   10                  15

Ser Ala Val Gly Phe Gly Ala Ser Pro Leu Gly Ser Val Phe Gly Pro
            20                  25                  30

Val Ala Glu Asp Asp Ala Val Ala Thr Val Arg Glu Ala Phe Arg Leu
        35                  40                  45

Gly Ile Asn Phe Phe Asp Thr Ser Pro Tyr Tyr Gly Gly Thr Leu Ser
    50                  55                  60

Glu Lys Met Leu Gly Lys Gly Leu Lys Ala Leu Gln Val Pro Arg Ser
65                  70                  75                  80

Asp Tyr Ile Val Ala Thr Lys Cys Gly Arg Tyr Lys Glu Gly Phe Asp
                85                  90                  95

Phe Ser Ala Glu Arg Val Arg Lys Ser Ile Asp Glu Ser Leu Glu Arg
            100                 105                 110

Leu Gln Leu Asp Tyr Val Asp Ile Leu His Cys His Asp Ile Glu Phe
        115                 120                 125

Gly Ser Leu Asp Gln Ile Val Ser Glu Thr Ile Pro Ala Leu Gln Lys
    130                 135                 140

Leu Lys Gln Glu Gly Lys Thr Arg Phe Ile Gly Ile Thr Gly Leu Pro
145                 150                 155                 160

Leu Asp Ile Phe Thr Tyr Val Leu Asp Arg Val Pro Pro Gly Thr Val
                165                 170                 175

Asp Val Ile Leu Ser Tyr Cys His Tyr Gly Val Asn Asp Ser Thr Leu
            180                 185                 190

Leu Asp Leu Leu Pro Tyr Leu Lys Ser Lys Gly Val Gly Val Ile Ser
        195                 200                 205

Ala Ser Pro Leu Ala Met Gly Leu Leu Thr Glu Gln Gly Pro Pro Glu
    210                 215                 220

Trp His Pro Ala Ser Pro Glu Leu Lys Ser Ala Ser Lys Ala Ala Val
225                 230                 235                 240

Ala His Cys Lys Ser Lys Gly Lys Lys Ile Thr Lys Leu Ala Leu Gln
                245                 250                 255

Tyr Ser Leu Ala Asn Lys Glu Ile Ser Ser Val Leu Val Gly Met Ser
            260                 265                 270

Ser Val Ser Gln Val Glu Glu Asn Val Ala Ala Val Thr Glu Leu Glu
        275                 280                 285

Ser Leu Gly Met Asp Gln Glu Thr Leu Ser Glu Val Glu Ala Ile Leu
    290                 295                 300

Glu Pro Val Lys Asn Leu Thr Trp Pro Ser Gly Ile His Gln Asn
305                 310                 315


<210> 12
<211> 960
<212> DNA
<213> Arabidopsis thaliana

<400> 12
atgacgaaaa tagagcttcg agctttgggg aacacagggc ttaaggttag cgccgttggt 60
tttggtgcct ctccgctcgg aagtgtcttc ggtccagtcg ccgaagatga tgccgtcgcc 120
accgtgcgcg aggctttccg tctcggtatc aacttcttcg acacctcccc gtattatgga 180
ggaacactgt ctgagaaaat gcttggtaag ggactaaagg ctttgcaagt ccctagaagt 240
gactacattg tggctactaa gtgtggtaga tataaagaag gttttgattt cagtgctgag 300
agagtaagaa agagtattga cgagagcttg gagaggcttc agcttgatta tgttgacata 360
cttcattgcc atgacattga gttcgggtct cttgatcaga ttgtgagtga aacaattcct 420
gctcttcaga aactgaaaca agaggggaag acccggttca ttggtatcac tggtcttccg 480
ttagatattt tcacttatgt tcttgatcga gtgcctccag ggactgtcga tgtgatattg 540
tcatactgtc attacggcgt taatgattcg acgttgctgg atttactacc ttacttgaag 600
agcaaaggtg tgggtgtgat aagtgcttct ccattagcaa tgggcctcct tacagaacaa 660
ggtcctcctg aatggcaccc tgcttcccct gagctcaagt ctgcaagcaa agccgcagtt 720
gctcactgca aatcaaaggg caagaagatc acaaagttag ctctgcaata cagtttagca 780
aacaaggaga tttcgtcggt gttggttggg atgagctctg tctcacaggt agaagaaaat 840
gttgcagcag ttacagagct tgaaagtctg gggatggatc aagaaactct gtctgaggtt 900
gaagctattc tcgagcctgt aaagaatctg acatggccaa gtggaatcca tcagaactaa 960

<210> 13
<211> 18
<212> PRT
<213> Artificial Sequence

<220>
<223> Description of Artificial Sequence: motif I of
      aldo-keto reductase superfamily

<220>
<221> VARIANT
<222> (2)
<223> Xaa = any amino acid

<220>
<221> VARIANT
<222> (4)..(5)
<223> Xaa = any amino acid

<220>
<221> VARIANT
<222> (7)
<223> Xaa = any amino acid

<220>
<221> VARIANT
<222> (9)..(13)
<223> Xaa = any amino acid

<220>
<221> VARIANT
<222> (15)..(17)
<223> Xaa = any amino acid

<400> 13
Gly Xaa Arg Xaa Xaa Asp Xaa Ala Xaa Xaa Xaa Xaa Xaa Glu Xaa Xaa
  1               5                  10                  15

Xaa Gly

<210> 14
<211> 30
<212> DNA
<213> Artificial Sequence

<220>
<223> Description of Artificial Sequence: Forward PCR
      Primer for L-galactono-1,4-lactone dehydrogenase
      from A. thaliana

<400> 14

caagaaggcc taaatgttcc gttacgctcc                                    30


<210> 15
<211> 30
<212> DNA
<213> Artificial Sequence

<220>
<223> Description of Artificial Sequence: Reverse PCR
      Primer for L-galactono-1,4-lactone dehydrogenase
      from A. thaliana

<400> 15
atgggccctt aagcagtggt ggagactggg                                    30


<210> 16
<211> 26
<212> DNA
<213> Artificial Sequence

<220>
<223> Description of Artificial Sequence: Forward PCR
      Primer for L-gulono-1,4-lactone oxidase from R.
      norvegicus

<400> 16
tgaggggtca gggtggtttg tttcca                                        26


<210> 17
<211> 28
<212> DNA
<213> Artificial Sequence

<220>
<223> Description of Artificial Sequence: Reverse PCR
      Primer for L-gulono-1,4-lactone oxidase from R.
      norvegicus

<400> 17
tggaatcatg gtccatgggt acaaaggg                                      28


<210> 18
<211> 22
<212> DNA
<213> Artificial Sequence

<220>
<223> Description of Artificial Sequence: Forward PCR
      Primer for D-arabinono-1,4-lactone oxidase from S.
      cerevisiae

<400> 18
tttcaccata tgtctactat cc                                                22

<210> 19
<211> 22
<212> DNA
<213> Artificial Sequence

<220>
<223> Description of Artificial Sequence: Reverse PCR
      Primer for D-arabinono-1,4-lactone oxidase from S.
      cerevisiae

<400> 19
aaggatccta gtcggacaac tc                                                22

<210> 20
<211> 344
<212> PRT
<213> Saccharomyces cerevisiae

<400> 20
Met Ser Ser Ser Val Ala Ser Thr Glu Asn Ile Val Glu Asn Met Leu
  1               5                  10                  15

His Pro Lys Thr Thr Glu Ile Tyr Phe Ser Leu Asn Asn Gly Val Arg
             20                  25                  30

Ile Pro Ala Leu Gly Leu Gly Thr Ala Asn Pro His Glu Lys Leu Ala
         35                  40                  45

Glu Thr Lys Gln Ala Val Lys Ala Ala Ile Lys Ala Gly Tyr Arg His
     50                  55                  60

Ile Asp Thr Ala Trp Ala Tyr Glu Thr Glu Pro Phe Val Gly Glu Ala
 65                  70                  75                  80

Ile Lys Glu Leu Leu Glu Asp Gly Ser Ile Lys Arg Glu Asp Leu Phe
                 85                  90                  95

Ile Thr Thr Lys Val Trp Pro Val Leu Trp Asp Glu Val Asp Arg Ser
            100                 105                 110

Leu Asn Glu Ser Leu Lys Ala Leu Gly Leu Glu Tyr Val Asp Leu Leu
        115                 120                 125

Leu Gln His Trp Pro Leu Cys Phe Glu Lys Ile Lys Asp Pro Lys Gly
    130                 135                 140

Ile Ser Gly Leu Val Lys Thr Pro Val Asp Asp Ser Gly Lys Thr Met
145                 150                 155                 160

Tyr Ala Ala Asp Gly Asp Tyr Leu Glu Thr Tyr Lys Gln Leu Glu Lys
                165                 170                 175

Ile Tyr Leu Asp Pro Asn Asp His Arg Val Arg Ala Ile Gly Val Ser
            180                 185                 190

Asn Phe Ser Ile Glu Tyr Leu Glu Arg Leu Ile Lys Glu Cys Arg Val
        195                 200                 205

Lys Pro Thr Val Asn Gln Val Glu Thr His Pro His Leu Pro Gln Met
    210                 215                 220

Glu Leu Arg Lys Phe Cys Phe Met His Asp Ile Leu Leu Thr Ala Tyr
225                 230                 235                 240

Ser Pro Leu Gly Ser His Gly Ala Pro Asn Leu Lys Ile Pro Leu Val
                245                 250                 255

Lys Lys Leu Ala Glu Lys Tyr Asn Val Thr Gly Asn Asp Leu Leu Ile
            260                 265                 270

Ser Tyr His Ile Arg Gln Gly Thr Ile Val Ile Pro Arg Ser Leu Asn
        275                 280                 285

Pro Val Arg Ile Ser Ser Ser Ile Glu Phe Ala Ser Leu Thr Lys Asp
    290                 295                 300

Glu Leu Gln Glu Leu Asn Asp Phe Gly Glu Lys Tyr Pro Val Arg Phe
305                 310                 315                 320

Ile Asp Glu Pro Phe Ala Ala Ile Leu Pro Glu Phe Thr Gly Asn Gly
                325                 330                 335

Pro Asn Leu Asp Asn Leu Lys Tyr
            340

<210> 21
<211> 1509
<212> DNA
<213> Saccharomyces cerevisiae

<400> 21
   taacaatttc gtttactgaa aatgctacta gtatataatc attaagtatc taactatcac 60
   tcaataaaaa tattatagat cgcttaaaaa ctcgtttatt gccgattata aatccaccaa 120
   aagccgctct acccttacct ccgcctggaa aaattataat atataaagtg agcctcgtaa 180
   tacaggggta aaaaggaaag agggggatat caagcatctg gacttatttg cactatctcc 240
   gccttcaatt gataaaagcg tcttgatttt aatcaactgc tatcatgtct tcttcagtag 300
   cctcaaccga aaacatagtc gaaaatatgt tgcatccaaa gactacagaa atatactttt 360
   cactcaacaa tggtgttcgt atcccagcac tgggtttggg gacagcaaat cctcacgaaa 420
   agttagctga aacaaaacaa gccgtaaaag ctgcaatcaa agctggatac aggcacattg 480
   atactgcttg ggcctacgag acagagccat tcgtaggtga agccatcaag gagttattag 540
   aagatggatc tatcaaaagg gaggatcttt tcataaccac aaaagtgtgg ccggttctat 600
   gggacgaagt ggacagatca ttgaatgaat ctttgaaagc tttaggcttg gaatacgtcg 660
   acttgctctt gcaacattgg ccgctatgtt ttgaaaagat taaggaccct aaggggatca 720
   gcggactggt gaagactccg gttgatgatt ctggaaaaac aatgtatgct gccgacggtg 780
   actatttaga aacttacaag caattggaaa aaatttacct tgatcctaac gatcatcgtg 840
   tgagagccat tggtgtctca aatttttcca ttgagtattt ggaacgtctc attaaggaat 900
   gcagagttaa gccaacggtg aaccaagtgg aaactcaccc tcacttacca caaatggaac 960
   taagaaagtt ctgctttatg cacgacattc tgttaacagc atactcacca ttaggttccc 1020
   atggcgcacc aaacttgaaa atcccactag tgaaaaagct tgccgaaaag tacaatgtca 1080
   caggaaatga cttgctaatt tcttaccata ttagacaagg cactatcgta attccgagat 1140
   ccttgaatcc agttaggatt tcctcgagta ttgaattcgc atctttgaca aaggatgaat 1200
   tacaagagtt gaacgacttc ggtgaaaaat acccagtgag attcatcgat gagccatttg 1260
   cagccatcct tccagagttt actggtaacg gaccaaactt ggacaattta aagtattaag 1320
   acaacgactt tattttcact ttatttagtt cgcttcttaa tcttgtcaaa aacaagatat 1380
   tgtgtaatcg cctcaagtaa acaatatgtt tttcatacgt gatttgaagt ttttaagtat 1440
   ctgaaataca tacgcgcgcg tatgcatatg tattagttaa attactcgaa tgtcctttat 1500
   ataatatta 1509

<210> 22
<211> 23
<212> DNA
<213> Artificial Sequence

<220>
<223> Description of Artificial Sequence: Forward PCR
      Primer for L-galactose dehydrogenase from A.
      thaliana

<400> 22
atgacgaaaa tagagcttcg agc 23

<210> 23
<211> 24
<212> DNA
<213> Artificial Sequence

<220>
<223> Description of Artificial Sequence: Reverse PCR
      Primer for L-galactose dehydrogenase from A.
      thaliana

<400> 23
ttagttctga tggattccac ttgg 24

<210> 24
<211> 24
<212> DNA
<213> Artificial Sequence

<220>
<223> Description of Artificial Sequence: Saccharomyces
      cerevisiae

<400> 24
atgtcttctt cagtagcctc aacc                                              24


<210> 25
<211> 29
<212> DNA
<213> Artificial Sequence

<220>
<223> Description of Artificial Sequence: Reverse PCR
      Primer for D-arabinose dehydrogenase from S.
      cerevisiae

<400> 25
ttaatacttt aaattgtcca agtttggtc                                         29


<210> 26
<211> 4
<212> PRT
<213> Artificial Sequence

<220>
<221> VARIANT
<222> (2)..(3)
<223> Xaa = any amino acid

<220>
<223> Description of Artificial Sequence: motif II of
      aldo-keto reductase superfamily

<400> 26
Gly Xaa Xaa Asn
  1


<210> 27
<211> 23
<212> DNA
<213> Artificial Sequence

<220>
<223> Description of Artificial Sequence: Forward PCR
      Primer for aldonolactonase from Z. mobilis

<400> 27
atgaccactg gtcgtatgtc tcg                                               23


<210> 28
<211> 24
<212> DNA

<213> Artificial Sequence

<220>
<223> Description of Artificial Sequence: Reverse PCR
      Primer for aldonolactonase from Z. mobilis

<400> 28
ttaccagaaa ataagaccca agca                                        24


<210> 29
<211> 320
<212> PRT
<213> Zymomonas mobilis

<400> 29
Met Thr Thr Gly Arg Met Ser Arg Arg Glu Cys Leu Ser Ala Ala Val
 1               5                  10                  15

Met Val Pro Ile Ala Ala Met Thr Ala Thr Ala Thr Ile Thr Gly Ser
            20                  25                  30

Ala Glu Ala Ala Lys Asn Asn Met Asn Gly Ser Thr Ile Gly Lys Ile
        35                  40                  45

Thr Lys Phe Ser Pro Arg Leu Asp Ala Ile Leu Asp Val Ser Thr Pro
    50                  55                  60

Ile Glu Val Ile Ala Ser Asp Ile Gln Trp Ser Glu Gly Pro Val Trp
65                  70                  75                  80

Val Lys Asn Gly Asn Phe Leu Leu Phe Ser Asp Pro Pro Ala Asn Ile
                85                  90                  95

Met Arg Lys Trp Thr Pro Asp Ala Gly Val Ser Ile Phe Leu Lys Pro
            100                 105                 110

Ser Gly His Ala Glu Pro Ile Pro Ala Gly Gln Phe Arg Glu Pro Gly
        115                 120                 125

Ser Asn Gly Met Lys Val Gly Pro Asp Gly Lys Ile Trp Val Ala Asp
    130                 135                 140

Ser Gly Thr Arg Ala Ile Met Lys Val Asp Pro Val Thr Arg Gln Arg
145                 150                 155                 160

Ser Val Val Asp Asn Tyr Lys Gly Lys Arg Phe Asn Ser Pro Asn Asp
                165                 170                 175

Leu Phe Phe Ser Lys Ser Gly Ala Val Tyr Phe Thr Asp Pro Pro Tyr
            180                 185                 190

Gly Leu Thr Asn Leu Asp Glu Ser Asp Ile Lys Glu Met Asn Tyr Asn
        195                 200                 205

Gly Val Phe Arg Leu Ser Pro Asp Gly Arg Leu Asp Leu Ile Glu Ala
    210                 215                 220

Gly Leu Ser Arg Pro Asn Gly Leu Ala Leu Ser Pro Asp Glu Thr Lys
225                 230                 235                 240

Leu Tyr Val Ser Asn Ser Asp Arg Ala Ser Pro Asn Ile Trp Val Tyr
                245                 250                 255

Ser Leu Asp Ser Asn Gly Leu Pro Thr Ser Arg Thr Leu Leu Arg Asn
            260                 265                 270

Phe Arg Lys Glu Tyr Phe Asp Gln Gly Leu Ala Gly Leu Pro Asp Gly
        275                 280                 285

Met Asn Ile Asp Lys Gln Gly Asn Leu Phe Ala Ser Ala Pro Gly Gly
    290                 295                 300

Ile Tyr Ile Phe Ala Pro Asp Gly Glu Cys Leu Gly Leu Ile Phe Trp
305                 310                 315                 320

<210> 30
<211> 963
<212> DNA
<213> Zymomonas mobilis

<400> 30
   atgaccactg gtcgtatgtc tcgtcgagaa tgcctttccg cagctgttat ggtgcctatc 60
   gccgctatga cagcaaccgc cactattaca ggatcagctg aggctgcaaa gaataatatg 120
   aacggatcta ccatcggtaa gattaccaaa ttttcacctc gccttgatgc tattttagat 180
   gtctctaccc caattgaggt cattgcatct gatattcaat ggtcagaagg ccctgtatgg 240
   gtcaaaaatg gcaatttcct gctgtttagt gatccgcccg ccaatattat gcgtaaatgg 300
   acgcctgatg ccggtgtcag catcttcctg aaaccttcag gtcatgccga acccattccg 360
   gccggtcagt tccgtgaacc gggttccaac ggtatgaaag ttggccccga tggcaaaatc 420
   tgggttgcag acagtggcac gcgcgcgatc atgaaagtgg atccggtcac ccgtcagcgc 480
   agtgttgtcg ataactataa aggcaaacgc tttaacagcc cgaatgacct cttcttctcg 540
   aagtcaggcg ctgtttattt caccgatccg ccctatggtc tgaccaatct tgatgaatcc 600
   gacatcaaag agatgaacta caatggtgtg ttccgtctct ctcccgatgg tcgtttagat 660
   ttgatcgaag ctggtcttag ccgcccgaat ggtttggctc tgtccccgga tgaaacgaaa 720
   ctttatgttt ctaactcaga ccgcgccagc ccgaatatct gggtttattc tttggatagc 780
   aatggcttgc caaccagccg gactttgctg cggaacttcc gcaaggaata tttcgatcag 840
   ggtctcgccg gtcttcctga tggtatgaat atcgacaaac agggcaatct ttttgcttct 900
   gctccgggcg gtatctatat ttttgcacca gatggcgaat gcttgggtct tattttctgg 960
   taa                                                                963

## Description

### BACKGROUND OF THE INVENTION

### 1. Field of the Invention

The present invention relates generally to the field of ascorbic acid production. More particularly, it relates to a process for the production of L-ascorbic acid from yeast, including recombinant yeast.

### 2. Description of Related Art

L-ascorbic acid (Vitamin C) is a powerful water-soluble antioxidant that is vital for growth and maintenance of all tissue types in humans. (Padh H., 1990, Biochem. Cell Biol. 68, 1166-1173) One important role of ascorbic acid is its involvement in the production of collagen, an essential cellular component for connective tissues, muscles, tendons, bones, teeth and skin. Collagen is also required for the repair of blood vessels, bruises, and broken bones. Ascorbic acid helps regulate blood pressure, contributes to reduced cholesterol levels, and aids in the removal of cholesterol deposits from arterial walls. Ascorbic acid also aids in the metabolization of folic acid, regulates the uptake of iron, and is required for the conversion of the amino acids L-tyrosine and L-phenylalanine into noradrenaline. The conversion of tryptophan into seratonin, the neurohormone responsible for sleep, pain control, and well-being, also requires adequate supplies of ascorbic acid.

A deficiency of L-ascorbic acid can impair the production of collagen and lead to joint pain, anemia, nervousness and retarded growth. Other effects are reduced immune response and increased susceptibility to infections. The most extreme form of ascorbic acid deficiency is scurvy, a condition evidenced by swelling of the joints, bleeding gums, and the hemorrhaging of capillaries below the surface of the skin. If left untreated, scurvy is fatal.

Although intestines easily absorb ascorbic acid, it is excreted to the urine within two to four hours of ingestion. Therefore, it cannot be stored in the body. L-ascorbic acid is produced in all higher plants and in the liver or kidney of most higher animals, but not humans, bats, some birds and a variety of fishes. Therefore, humans must have access to sufficient amounts of ascorbic acid from adequate dietary sources or supplements in order to maintain optimal health.

Food sources of ascorbic acid include citrus fruits, potatoes, peppers, green leafy vegetables, tomatoes, and berries. Ascorbic acid is also commercially available as a supplement in forms such as pills, tablets, powders, wafers, and syrups.

L-Ascorbic acid is approved for use as a dietary supplement and chemical preservative by the U.S. Food and Drug Administration and is on the FDA's list of substances generally recognized as safe. L-Ascorbic acid may be used in soft drinks as an antioxidant for flavor ingredients, in meat and meat-containing products, for curing and pickling, in flour to improve baking quality, in beer as a stabilizer, in fats and oils as an antioxidant, and in a wide variety of foods for ascorbic acid enrichment. L-Ascorbic acid may also find use in stain removers, hair-care products, plastics manufacture, photography, and water treatment.

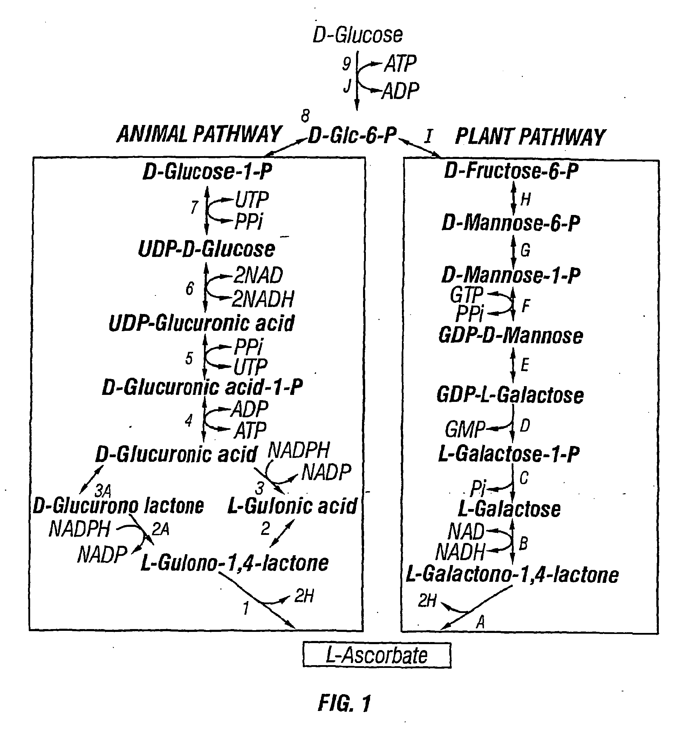

The enzymes of the biosynthetic pathways leading to ascorbic acid have not been identified yet to completion. Current understanding of the physiological pathways in plants and animals is shown in Fig. 1.

In animals, D-glucose serves as the first precursor and the last step is catalyzed by a microsomal L-gulono-1,4-lactone oxidase. The enzyme has been isolated and characterized from different sources. The gene from rat has been cloned and sequenced (Koshizaka T. et al., 1988, J. Biol. Chem. 263, 1619-1621).

Two discrete pathways have been reported for ascorbic acid synthesis in plants. In one pathway, L-ascorbic acid is synthesized from D-glucose via L-sorbosone (Loewus M.W. et al., 1990, Plant. Physiol. 94, 1492-1495). Current evidence suggests that the main physiological pathway proceeds from D-glucose via L-galactose and L-galactono-1,4-lactone to L-ascorbic acid (Wheeler G.L. et al. 1998, Nature, 393, 365-369). The last two steps are catalyzed by the enzymes L-galactose dehydrogenase and L-galactono-1,4-lactone dehydrogenase. Also in this case, the last enzyme has been isolated and characterized, and the gene from *Brassica oleracea* has been cloned and sequenced (∅stergaard J. et al. 1997, J. Biol. Chem., 272, 30009-30016).

For use as a dietary supplement, ascorbic acid can be isolated from natural sources or synthesized chemically by the oxidation of L-sorbose as in variations of the Reichstein process (U.S. Pat. No. 2,265,121).

It remains desirable to have methods for the production of ascorbic acid by convenient processes. Two main requirements in the production of ascorbic acid are that the synthesis should be enantioselective, because only the L-enantiomer of ascorbic acid is biologically active, and that the environment of the final steps of the process should be non-oxidative, because ascorbic acid is very easily oxidized.

One possible approach is the production of L-ascorbic acid from microorganisms. Microorganisms can be easily grown on an industrial scale. Although the production of L-ascorbic acid from microorganisms and fungi has been reported in the past, recent evidence proves that L-ascorbic acid analogues, and not L-ascorbic acid, are produced (Huh W.K. et al. 1998, Mol. Microbiol. 30, 895-903)(Hancock R.D. et al., 2000, FEMS Microbiol. Let. 186, 245-250)(Dumbrava V.A. et al. 1987, BBA 926, 331-338). In yeasts *(Candida* and *Saccharomyces* species), the production of erythroascorbic acid has been reported (Huh W.K. et al., 1994, Eur. J. Biochem, 225, 1073-1079)(Huh W.K. et al., 1998, Mol. Microbiol. 30, 4, 895-903). In such yeasts, a physiological pathway has been proposed proceeding from D-glucose via D-arabinose and D-arabinono-1,4-lactone to erythroascorbic acid (Kim S.T. et al., 1996, BBA, 1297, 1-8). The enzymes D-arabinose dehydrogenase and D-arabinono-1,4-lactone oxidase from *Candida albicans* as well as *S. cerevisiae* have been characterized. Interestingly, L-galactose and L-galactono-1,4-lactone are substrates for these activities *in vitro.*

*In vivo* production of L-ascorbic acid has been obtained by feeding L-galactono-1,4-lactone to wild-type *Candida* cells (International Patent Application WO85/01745). Recently it has been shown that wild-type *S. cerevisiae* cells accumulated L-ascorbic acid intracellularly when incubated with L-galactose, L-galactono-1,4-lactone, or L-gulono-1,4-lactone (Hancock et al., 2000, FEMS Microbiol. Lett. 186, 245-250)(Spickett C.M. et al., 2000, Free Rad. Biol. Med. 28, 183-192).

Wild-type *Candida* cells incubated with L-galactono-1,4-lactone accumulate L-ascorbic acid in the medium, suggesting that this yeast has a biological mechanism for the release of L-ascorbic acid accumulated intracellularly; indeed, L-ascorbic acid is a complex molecule and it is scientifically reasonable that its accumulation in the medium is not related to a simple diffusion process, but should depend on facilitated or active transport. This conclusion is supported by the identification and characterization of L-ascorbic acid transporters in higher eukaryotic (mammalian) cells (Daruwala R. et al., 1999, FEBS Letters. 460, 480-484). However, L-ascorbate transporters have not been described among the yeast genera. Nevertheless, while *Candida* cells growing in media containing L-galactono-1,4-lactone accumulate L-ascorbic acid in the medium, accumulation in the medium of L-ascorbic acid from *S*. *cerevisiae* cells incubated in presence of ascorbic acid precursor has, surprisingly, never been described.

A desirable method for the large-scale production of ascorbic acid comprises the use of genetically engineered microorganisms (i.e., recombinant microorganisms). Both prokaryotic and eukaryotic microorganisms are today easily and successfully used for the production of heterologous proteins as well as for the production of heterologous metabolites. Among prokaryotes, *Escherichia coli* and *Bacillus subtilis* are often used. Among eukaryotes, the yeasts *S. cerevisiae* and *Kluyveromyces lactis* are often used. Despite the great success of these hosts, only one example has been described for the production of L-ascorbic acid by transformed microbial cells. Since only eukaryotic cells are natural L-ascorbic acid producers, it is even more surprising that only a prokaryotic transformed microbial host has been described to lead to the intracellular accumulation of L-ascorbic acid. Lee et al. (Appl. Environment. Microbiol., 1999, 65, 4685-4687), showed that the cloning of the *S. cerevisiae* gene encoding D-arabinono-1,4-lactone oxidase into *E. coli* allows the production of L-ascorbic acid from *E*. *coli* incubated with L-galactono-1,4-lactone. Accumulation of L-ascorbic acid was observed only at the intracellular level.

No experimental data have been described in the literature about the production of L-ascorbic acid from transformed eukaryotic microorganisms. Østergaard et al. cloned the gene encoding L-galactono-1,4-lactone dehydrogenase from cauliflower in the yeast S. cerevisiae (J. Biol. Chem., 1997, 272, 30009-30016). While, *in vitro,* the authors found L-galactono-1,4-lactone dehydrogenase activity in the yeast cell extract (cytochrome c assay, see Østergaard et al.), no production ofL-ascorbic acid was proven *in vivo.*

Berry et al., International Patent Appln. WO 99/64618 discuss the potential use of the plant biosynthetic pathway of ascorbic acid; special emphasis is given to the activity catalyzing the conversion of GDP-D-mannose to GDP-L-galactose. However, characterization of the enzyme catalyzing this step has not been presented in detail. An overexpressed *E. coli* homologue turned out to be inactive.

Smirnoff et al., WO 99/33995, discuss the use of L-galactose dehydrogenase for production of ascorbic acid. The enzyme was purified from pea seedlings and the N-terminal protein sequence was determined. The complete sequence is not known and has not yet been reported. The L-galactose dehydrogenase enzyme partial sequence was 72% identical to amino acids 5-22 of an unidentified putative coding sequence from *Arabidopsis thaliana,* accession no. 3549669.

Roland et al., U.S. Patents Nos. 4,595,659 and 4,916,068, discuss the use of non-recombinant *Candida* strains to convert L-galactonic substrates to L-ascorbic acid. Roland et al. described the responsible enzyme as L-galactono-1,4-lactone oxidase.

Kumar, WO 00/34502, discusses the production of L-ascorbic acid in *Candida blankii* and *Cryptococcus dimennae* yeast capable of using 2-keto-L-gulonic acid as a sole carbon source in the production. Kumar specifically excludes the production from yeast by a pathway involving L-galactonolactone oxidase or by conversion of L-galactonic precursors.

It remains desirable to have methods for the production of ascorbic acid by a convenient fermentation process.

### SUMMARY OF THE INVENTION

In one embodiment, this invention relates to a method of generating ascorbic acid or a salt thereof, comprising (i) culturing a *Kluyveromyces* spp. or a *Zygosaccharomyces* spp. yeast in a medium comprising at least one ascorbic acid precursor, thereby forming ascorbic acid or a salt thereof, and (ii) isolating the ascorbic acid or salt thereof.

In a second embodiment, the present invention relates to a method of generating ascorbic acid or a salt thereof, comprising (i) culturing a recombinant yeast in a medium comprising an ascorbic acid precursor, thereby forming ascorbic acid or a salt thereof, and (ii) isolating the ascorbic acid or a salt thereof. Preferably, the recombinant yeast accumulates ascorbic acid in the medium at a level greater than the background. Also preferably, the recombinant yeast produces ascorbic acid at a yield greater than about 35% from the precursor.

In a third embodiment, the present invention relates to a method of stabilizing ascorbic acid or a salt thereof added to the medium or produced by the yeast, comprising culturing a yeast in the medium.

Another embodiment is directed to a recombinant yeast that is functionally transformed with at least one coding region encoding a protein having an enzyme activity selected from the group consisting of L-galactose dehydrogenase (LGDH), L-galactono-1,4-lactone dehydrogenase (AGD), D-arabinose dehydrogenase (ARA), D-arabinono-1,4-lactone oxidase (ALO), L-gulono-1,4-lactone oxidase (GLO), and aldonolactonase (AL). Preferably, the recombinant yeast functionally transformed with the coding region can be capable of at least one of (a) converting to ascorbic acid at least about 25% ascorbic acid precursor when the yeast is cultured in a culture medium comprising at least one ascorbic acid precursor or (b) producing at least about 20 mg ascorbic acid/L medium when the yeast is cultured in a medium comprising at least one ascorbic acid precursor. The recombinant yeast preferably belongs to the species *S*. *cerevisiae, K. lactis,* or *Z. bailii.*

Yet another embodiment is directed to a method of generating ascorbic acid or a salt thereof by culturing a recombinant yeast in a medium comprising at least one ascorbic acid precursor, thereby forming ascorbic acid or a salt thereof, and isolating the ascorbic acid or salt thereof. Preferably, the recombinant yeast is capable of at least one of (a) converting at least about 25% of the ascorbic acid precursor into ascorbic acid or a salt thereof or (b) producing at least about 20 mg ascorbic acid/ l medium in the culture.

The present invention provides methods for the production of ascorbic acid by a convenient fermentation process.

### DESCRIPTION OF THE DRAWINGS

**Figure 1** provides a schematic representation of the current understanding of the physiological biosynthetic pathways leading from D-glucose to L-ascorbic acid in plants or animals, respectively. The following enzymes are involved: A, L-galactono-1,4-lactone dehydrogenase (1.3.2.3); B, L-galactose dehydrogenase; C, sugar phosphatase (3.1.3.23, putative); D, hydrolase (putative); E, GDP-mannose-3,5-epimerase (5.1.3.18); F, mannose-1-phosphate guanylyltransferase (2.7.7.22); G, phosphomannomutase (5.4.2.8); H, mannose-6-phosphate isomerase (5.3.1.8); I, glucose-6-phosphate isomerase (5.3.1.9); J, hexokinase (2.7.1.1); 1, L-gulono-1,4-lactone oxidase (1.1.3.8); 2, aldonolactonase (3.1.1.17); 2a, glucurono lactone reductase (1.1.1.20); 3, D-glucuronate reductase (1.1.1.19); 3a, uronolactonase (3.1.1.19) or spontaneous; 4, D-glucurono kinase (2.7.1.43); 5, glucuronate-1-phosphate uridylyltransferase (2.7.7.44); 6, UDP-D-glucose dehydrogenase (1.1.1.22); 7, UTP-glucose-1-phosphate uridylyltransferase (2.7.7.9); 8, phosphoglucomutase (5.4.2.2), 9, hexokinase (2.7.1.1). However, it has to be stressed that in the scope of the present invention to produce L-ascorbic acid, the enzymes useful are not limited to the enzymes of the physiological pathways.

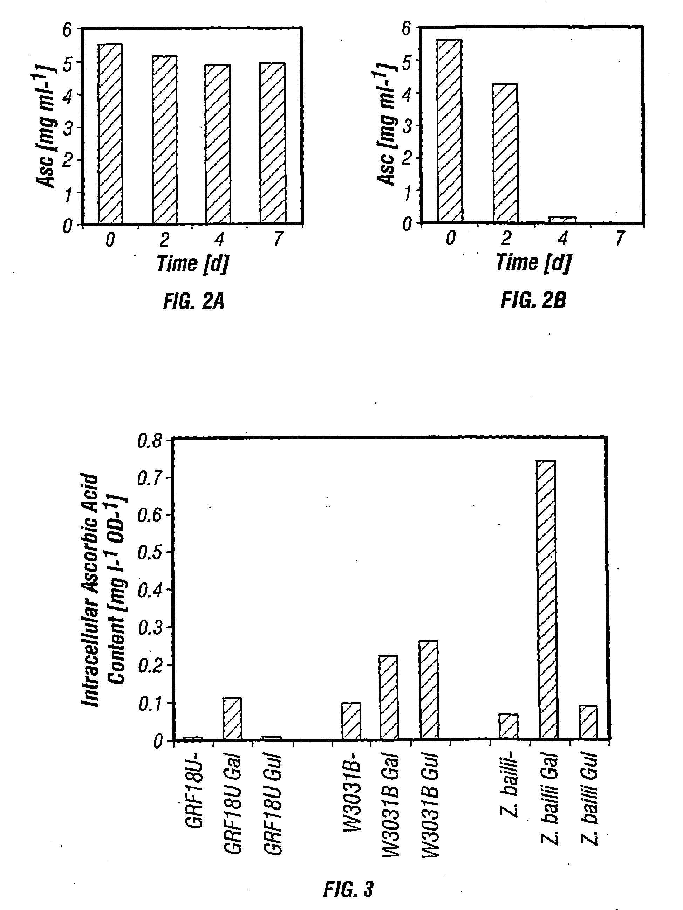

**Figure 2** shows the stability of ascorbic acid under culture conditions. Ascorbic acid was added to mineral medium (2% glucose, 0.67% YNB) and incubated under standard culture conditions for 7 days. The flask of panel A was inoculated at time 0 with *S. cerevisiae* GRF18U to an initial OD⁶⁶⁰ of 0.05, whereas the flask of panel B was kept sterile. Samples were taken at the indicated times and the ascorbic acid concentration was determined. Although the ascorbic acid was stable in this medium when viable yeast were present, it was completely degraded within 7 days in sterile medium. This demonstrates that incubation of viable yeast in a medium comprising ascorbic acid can be used as a method of stabilizing ascorbic acid.
**Figure 3** shows the endogenous ability of yeasts to convert the precursors L-galactono-1,4-lactone (Gal) or L-gulono-1,4-lactone (Gul) to ascorbic acid. Non-transformed yeast cells *(S. cerevisiae* GRF18U, W3031B and *Z bailii* ATCC 60483) were grown on mineral medium (2% glucose, 0.67% YNB) in the presence of 100 mM L-galactono-1,4-lactone or L-gulono-1,4-lactone, respectively, for 72 hr. (Initial OD⁶⁶⁰ was 0.05); "-" signifies that no precursor was added. While ascorbic acid was accumulated within the cell, no ascorbic acid could be detected in the culture broth.

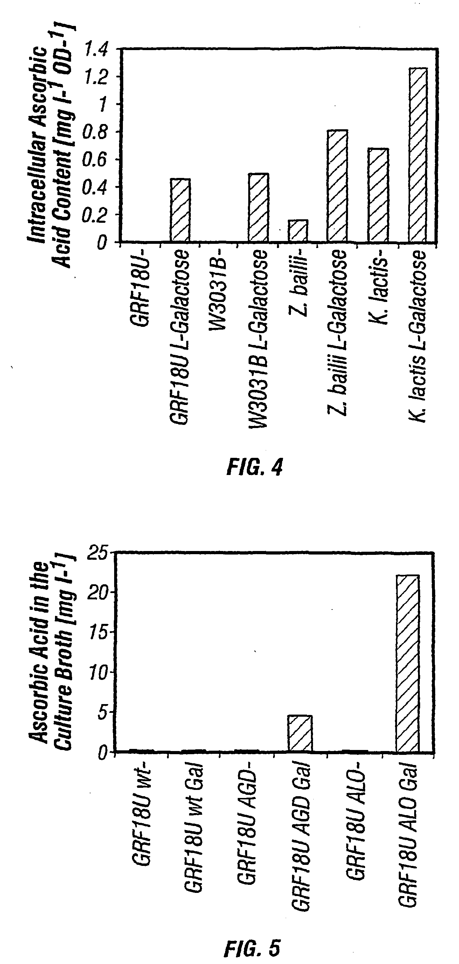

**Figure 4** shows the endogenous ability of yeasts to convert L-galactose to ascorbic acid. Non-transformed *S. cerevisiae* (GRF18U and W3031B), *Z*. *bailii* ATCC 60483 and *K. lactis* PM6-A were grown on mineral medium (2% glucose, 0.67% YNB) starting from an OD⁶⁶⁰ of 0.05 overnight. Then, 250 mg 1⁻¹ L-galactose were added and the cultures were kept under standard conditions for another 24 hr before the determination of ascorbic acid. All of these strains accumulated ascorbic acid intracellularly while no ascorbic acid was measurable in the culture broth except for *Z*. *bailii,* which accumulated a small amount. (It is believed the high background in *K. lactis* is due to erythroascorbic acid, naturally present in this yeast species at higher concentrations than seen in *S. cerevisiae*).
**Figure 5** shows the conversion of L-galactono-1,4-lactone to ascorbic acid by recombinant yeasts. *S*. *cerevisiae* GRF18U wt (control), or transformed with a coding region encoding AGD or ALO, respectively, were grown on mineral medium (2% glucose, 0.67% YNB) starting from an OD⁶⁶⁰ of 0.05 in the presence of 50 mM L-galactono-1,4-lactone (Gal) for 72 hr. While the control cells did not accumulate ascorbic acid in the culture medium, cells transformed with a coding region encoding AGD or ALO unexpectedly accumulated considerable amounts (i.e. greater than background levels) of ascorbic acid in the culture medium. No ascorbic acid was detected in cultures without the addition of L-galactono-1,4-lactone (marked -).

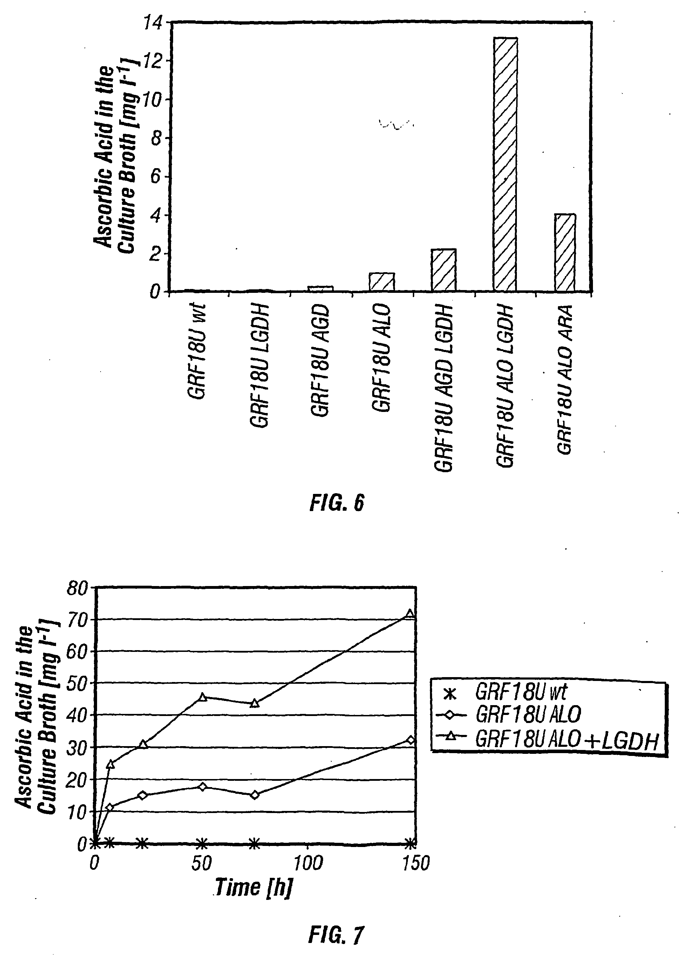

**Figure 6** shows the conversion of L-galactose to ascorbic acid by recombinant yeasts. *S. cerevisiae* GRF18U wt (control), transformed with a coding region or coding regions encoding LGDH; AGD; ALO; AGD and LGDH; ALO and LGDH; or ARA and ALO, respectively, were grown on mineral medium (2% glucose, 0.67% YNB) starting from an OD⁶⁶⁰ of 0.05 over night. Then 250 mg 1⁻¹ L-galactose were added and the cultures were kept under standard conditions for another 24 hr before the determination of ascorbic acid. The control cells or cells transformed with only a coding region encoding LGDH did not accumulate ascorbic acid in the culture medium. Cells transformed with coding regions encoding LGDH and either AGD or ALO, as well as cells transformed with coding regions encoding ARA and ALO, accumulated considerable amounts (i.e. greater than background levels) of ascorbic acid in the medium.
**Figure 7** shows the conversion of L-galactose to ascorbic acid in a high cell density culture of recombinant yeast. *S. cerevisiae* GRF18U wt (control) or GRF18U transformed with a coding region encoding ALO, or coding regions encoding LGDH and ALO, respectively, were grown on mineral medium (2% glucose, 0.67% YNB) starting from an OD⁶⁶⁰ of 0.05 overnight. At time 0 the cells were concentrated 10 times and 250 mg l⁻¹ L-galactose were added and the cultures were kept under standard conditions for 6 days. At the times indicated, samples were taken and the ascorbic acid concentration in the culture broth was measured. While the control cells did not accumulate ascorbic acid in the culture medium, cells transformed with a coding region encoding ALO alone or with coding regions encoding ALO and LGDH accumulated considerable amounts (i.e. greater than background levels) of ascorbic acid in the medium.

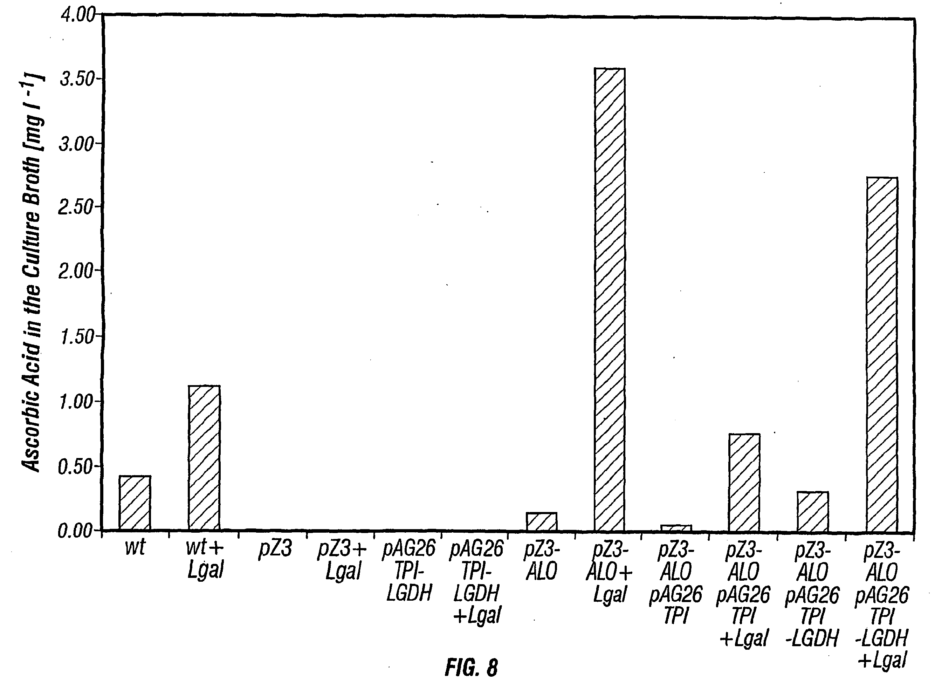

**Figure 8** shows the conversion of L-galactose to ascorbic acid by recombinant yeast. *Z. bailii* ATCC 36947 wt (control), *Z. bailii* transformed with empty vector (pZ3); a coding region encoding LGDH; a coding region encoding ALO; or coding regions encoding ALO and LGDH; respectively, were grown on mineral medium (2% glucose, 0.67% YNB) starting from an OD⁶⁶⁰ of 0.05 overnight in the presence of appropriate selective antibiotics (G418 for pZ3, hygromycin for pAG26TPI, or both). Then 250 mg l⁻¹ L-galactose were added and the cultures were kept under standard conditions for another 24 hr before the determination of ascorbic acid. Wt cells *Z. bailii* ATCC 36947 accumulated minor amounts of ascorbic acid in the medium upon incubation with L-galactose. Cells transformed with the empty vector or a vector comprising the coding region encoding LGDH did not accumulate ascorbic acid in the culture medium. Cells transformed with only the coding region encoding ALO, or with coding regions encoding LGDH and ALO accumulated considerable amounts (i.e. greater than background levels) of ascorbic acid in the medium. Expression of antibiotic resistance used for plasmid selection or the antibiotics used for such selection may interfere with the accumulation or determination of ascorbic acid in cultures. This correlates with the data, in that cells expressing ALO without expressing hygromycin resistance and without hygromycin in the medium accumulated more ascorbic acid than cells expressing ALO along with hygromycin resistance, which were grown in medium comprising hygromycin.

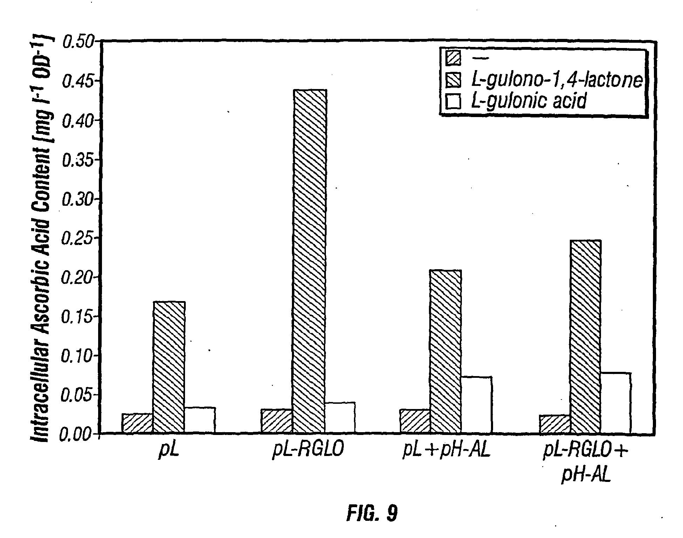

**Figure 9** shows the conversion of L-gulonic acid and L-gulono-1,4-lactone to ascorbic acid by recombinant *S. cerevisiae* GRF18U. The GRF18U cells were transformed with an empty plasmid (pL, control); plasmid pL-RGLO; plasmids pL and pH-AL; or plasmid pL-RGLO (RGLO is GLO isolated from *R*. *norvegicus)* and pH-AL, respectively. The recombinant yeast were grown on minimal medium (2% glucose, 0.67% YNB) starting from an OD⁶⁶⁰ of 0.05 over night. Then, 66 mM of L-gulonic acid or L-gulono-1,4-lactone was added. After 48 hours, the cells were harvested and the intracellular ascorbic acid concentration was determined. Ascorbic acid was not detected in the supernatant (not shown). Cells transformed with the coding region encoding AL accumulated ascorbic acid at greater than background levels with L-gulonic acid present as substrate. Cells transformed with RGLO only accumulated about three times the amount of ascorbic acid seen in the control (e.g., GRF 18U/pL) with L-gulono-1,4-lactone present as substrate.

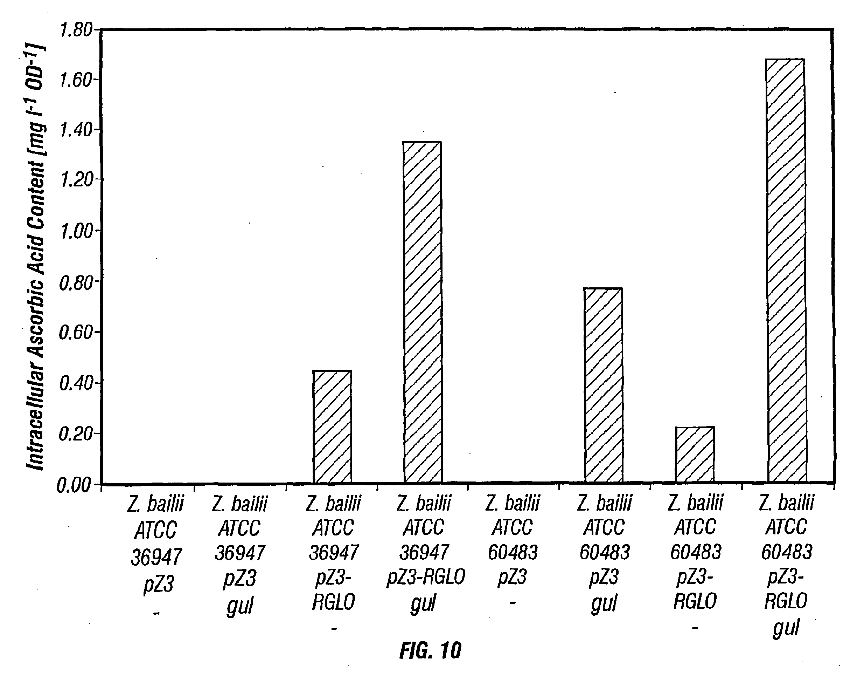

**Figure 10** shows the intracellular conversion of L-gulono-1,4-lactone (e.g., gul) to ascorbic acid in a culture of recombinant *Z. bailii. Z. bailii* ATCC 36947 and *Z. bailii* ATCC 60483 were transformed with either the empty *Z. bailii* expression plasmid pZ3 or with pZ3-RGLO (RGLO is GLO isolated from *R. norvegicus).* The yeast was grown on mineral medium (2% glucose, 0.67% YNB) starting from an OD⁶⁶⁰ of 0.05 overnight in presence of G418. Then 100 mM L-gulonic acid-1,4-lactone was added and the cultures were kept under standard conditions for another 48 hr before the determination of ascorbic acid. Both *Z. bailii* strains expressing RGLO produced relatively high intracellular levels of ascorbic acid in the presence of L-gulono-1,4-lactone.

### DESCRIPTION OF ILLUSTRATIVE EMBODIMENTS

In one embodiment, this invention relates to a method of generating ascorbic acid or a salt thereof, comprising (i) culturing a *Kluyveromyces* spp. or a *Zygosaccharomyces* spp. yeast in a medium comprising at least one ascorbic acid precursor, thereby forming ascorbic acid or a salt thereof, and (ii) isolating the ascorbic acid or a salt thereof. This method is based on the scientific observation that wild-type yeast of the genus *Kluyveromyces* or *Zygosaccharomyces* are capable of generating L-ascorbic acid when cultured in a medium containing an ascorbic acid pathway precursor. Preferably, the yeast is *Z. bailii* or *K. lactis.* More preferably, the yeast is *Z*. *bailii* ATCC 60483, *Z. bailii* ATCC 36947 or *K. lactis* PM6-7A.

The medium in which the yeast is cultured can be any medium known in the art to be suitable for this purpose. Culturing techniques and media are well known in the art. Typically, but it is not limited to, culturing is performed by aqueous fermentation in an appropriate vessel. Examples for a typical vessel for yeast fermentation comprise a shake flask or a bioreactor.

The medium comprises any component required for the growth of the yeast and one or more precursors for the production of ascorbic acid. Components for growth of the yeast and precursors for the production of ascorbic acid may or may be not identical.

The medium comprises a carbon source, such as glucose or other carbohydrates (such as sucrose, fructose, arabinose, lactose, galactose, maltose, raffinose, ethanol, methanol glycerol, or hydrolysates of vegetable matter, among others). Typically, the medium also comprises a nitrogen source, either organic or inorganic, and the medium may also comprise components such as amino acids; purines; pyrimidines; corn steep liquor; yeast extract; protein hydrolysates; water-soluble vitamins, such as B complex vitamins; or inorganic salts such as chlorides, hydrochlorides, phosphates, or sulfates of Ca, Mg, Na, K, Fe, Ni, Co, Cu, Mn, Mo, or Zn, among others. Further components known to one of ordinary skill in the art to be useful in yeast culturing or fermentation can also be included. The medium may or may be not buffered.

The medium also comprises an ascorbic acid precursor. The ascorbic acid precursor is any compound that, in the yeast, can be converted, either directly or through intermediate steps, into L-ascorbic acid. Ascorbic acid precursors can be selected from the group consisting of monosaccharides, oligosaccharides, polysaccharides, C₁-C₆ alcohols, C₂-C₆polyols, organic acids including amino acids and fatty acids, and C₅-C₆ lactones. The saccharides and organic acids, can comprise saccharides and organic acids that have been phosphorylated and/or modified with UDP or GDP. Preferably the ascorbic acid precursor is selected from the group consisting of trehalose; xylose; xylulose; raffinose; ethanol; maltose; glycerol; fructose; arabinose; D-glucose-6-P; D-glucose-1-P; UDP-D-glucose; UDP-glucuronic acid; D-glucuronic acid-1-P; D-glucuronic acid; D-glucurono lactone; L-gulonic acid; D-fructose-6-P; D-mannose-6-P; D-mannose-1-P; GDP-D-mannose; GDP-L-galactose; L-galactose-1-P; lactose; sucrose; starch; cellulose; citrate; methanol; formaldehyde; formate; methylamine; alanine; oleic acid; L-galactono-1,4-lactone; D-glucose; L-gulono-1,4-lactone; L-galactose; and L-gulonic acid, among others. More preferably the ascorbic acid precursor is selected from L-galactono-1,4-lactone; D-glucose; L-gulono-1,4-lactone; L-galactose; and L-gulonic acid. Two or more ascorbic acid precursors can also be used. Furthermore, in certain embodiments, the ascorbic acid precursor can also serve as a carbon source for culturing the yeast. During the course of the fermentation, the yeast converts the ascorbic acid precursor to L-ascorbic acid via one or more steps. The L-ascorbic acid produced by wild type *Kluyveromyces spp.* or *Zygosaccharomyces spp.* yeast can be accumulated within the yeast. A preferred medium comprises glucose, YNB (Yeast Nitrogen Base), and at least one of L-gulonic acid; L-galactono-1,4-lactone; L-gulono-1,4-lactone; or L-galactose.

After culturing has progressed for a sufficient length of time to produce a desired concentration of L-ascorbic acid in the yeast, the culture medium, or both, the L-ascorbic acid is isolated. "Isolated," as used herein to refer to ascorbic acid, means being brought to a state of greater purity by separation of ascorbic acid from at least one non-ascorbic acid component of the yeast or the medium. Preferably, the isolated ascorbic acid is at least about 95% pure, more preferably at least about 99% pure.

To isolate L-ascorbic acid from the yeast, the first step of isolation, after the yeast is separated from the medium, typically is lysing of the yeast by chemical or enzymatic treatment, treatment with glass beads, sonication, freeze/thaw cycling, or other known techniques. L-ascorbic acid can be purified from the membrane, protein, nucleic acid and other fractions of the yeast lysate by appropriate techniques, such as centrifugation, filtration, microfiltration, ultrafiltration, nanofiltration, liquid-liquid extraction, crystallization, enzymatic treatment with nuclease or protease, or chromatography, among others.

To isolate L-ascorbic acid accumulated in the medium, the isolation comprises purifying the ascorbic acid from the medium. Purification can be performed by known techniques, such as the use of an ion exchange resin, activated carbon, microfiltration, ultrafiltration, nanofiltration, liquid-liquid extraction, crystallization, or chromatography, among others.

L-ascorbic acid can be isolated from both the yeast and the medium.

If the yeast accumulates L-ascorbic acid in the medium during the culturing step, preferably the concentration of L-ascorbic acid is stabilized or allowed to increase. Still more preferably the L-ascorbic acid in the medium is stabilized by the presence of viable yeast.

In a second embodiment, the present invention relates to a method of generating ascorbic acid, comprising (i) culturing a recombinant yeast in a medium comprising at least one ascorbic acid precursor, thereby forming ascorbic acid, and (ii) isolating the ascorbic acid.

A "recombinant" yeast is a yeast that contains a nucleic acid sequence not naturally occurring in the yeast or an additional copy or copies of an endogenous nucleic acid sequence, wherein the nucleic acid sequence is introduced into the yeast or an ancestor cell thereof by human action. Recombinant DNA techniques are well known, such as in Sambrook et al., Molecular Cloning: A Laboratory Manual, Cold Spring Harbor Laboratory Press, which provides further information regarding various techniques known in the art and discussed herein. In this embodiment, a coding region of the homologous and/or heterologous gene is isolated from an organism, which possesses the gene. The organism can be a bacterium, a prokaryote, a eukaryote, a microorganism, a fungus, a plant, or an animal.

Genetic material comprising the coding region can be extracted from cells of the organism by any known technique. Thereafter, the coding region can be isolated by any appropriate technique. In one known technique, the coding region is isolated by, first, preparing a genomic DNA library or a cDNA library, and second, identifying the coding region in the genomic DNA library or cDNA library, such as by probing the library with a labeled nucleotide probe selected to be or presumed to be at least partially homologous with the coding region, determining whether expression of the coding region imparts a detectable phenotype to a library microorganism comprising the coding region, or amplifying the desired sequence by PCR. Other known techniques for isolating the coding region can also be used.

The recombinant yeast can belong to any known genus and species of yeast. Yeasts are described by N. J. W. Kreger-van Rij, "The Yeasts," Vol. 1 of Biology of Yeasts, Ch. 2, A. H. Rose and J. S. Harrison, Eds. Academic Press, London, 1987. For example, the yeast genus can be *Saccharomyces, Zygosaccharomyces, Candida, Hansenula, Kluyveromyces, Debaromyces, Nadsonia, Lipomyces, Torulopsis, Kloeckera, Pichia, Schizosaccharomyces, Trigonopsis, Brettanomyces, Cryptococcus, Trichosporon, Aureobasidium, Lipomyces, Phaffia, Rhodotorula, Yarrowia,* or *Schwanniomyces,* among others. *Saccharomyces, Zygosaccharomyces, Kluyveromyces* spp. are preferred. More preferably, the yeasts are *S*. *cerevisiae, Z. bailii and K. lactis.* Even more preferably, the yeast is *S. cerevisiae* strain GRF18U or W3031B, *Z. bailii* ATCC 60483, *Z. bailii* ATCC 36947 or *K. lactis* PM6-7A.

Preferably, a recombinant yeast of the present invention is not able to produce L-ascorbic acid from 2-keto-L-gulonic acid.

Preferably, the recombinant yeast comprises at least one coding region encoding an enzyme, such that the recombinant yeast, which has been functionally transformed, is capable of converting an ascorbic acid precursor to ascorbate in one or more steps.

The medium in which the recombinant yeast is cultured can be any medium known in the art to be suitable for this purpose. Culturing techniques and media are well known in the art. Typically, but it is not limited to, culturing is performed by aqueous fermentation in an appropriate vessel. Examples for a typical vessel for yeast fermentation comprise a shake flask or a bioreactor.

The medium comprises any component required for the growth of the yeast and one or more precursors for the production of ascorbic acid. Components for growth of the yeast and precursors for the production of ascorbic acid may or may be not identical, and suitable medium components and ascorbic acid precursors are described above. During the course of the fermentation, the ascorbic acid precursor is internalized by the recombinant yeast and converted, through one or more steps, into L-ascorbic acid. The L-ascorbic acid produced by the recombinant yeast can be contained within the yeast, or can be accumulated in the medium at greater than background levels.

After culturing has progressed for a sufficient length of time to produce a desired concentration of L-ascorbic acid in the yeast, the culture medium, or both, the L-ascorbic acid is isolated, as described above. L-ascorbic acid can be isolated from both the yeast and the medium. As explained above, if the yeast accumulates L-ascorbic acid in the medium during the culturing step, preferably the concentration of L-ascorbic acid is stabilized or allowed to increase. Still more preferably the L-ascorbic acid in the medium is stabilized by the presence of viable yeast.

In a preferred embodiment of the present invention, the one or more coding regions introduced into a recombinant yeast encode at least one enzyme selected from L-galactose dehydrogenase (LGDH), L-galactono-1,4-lactone dehydrogenase (AGD), D-arabinose dehydrogenase (ARA), D-arabinono-1,4-lactone oxidase (ALO), L-gulono-1,4-lactone oxidase (GLO) and aldonolactonase (AL). In one more preferred embodiment, the coding region of L-galactose dehydrogenase (LGDH), L-galactono-1,4-lactone dehydrogenase (AGD), D-arabinose dehydrogenase (ARA), D-arabinono-1,4-lactone oxidase (ALO), L-gulono-1,4-, lactone oxidase (GLO) or aldonolactonase (AL) is isolated from *A. thaliana, S. cerevisiae, Rattus norvegicus* or *Zymomonas mobilis.* It should be noted that the term "isolated," as used herein in reference to a nucleic acid sequence, refers to the ultimate source, not the immediate source, of the coding region. That is, a coding region is "isolated" from an organism if it encodes a protein sequence substantially identical to that of the same protein purified from cells of the organism. In even more preferred embodiments, the coding regions encoding LGDH and AGD are isolated from *A. thaliana,* the coding regions encoding ALO and ARA are isolated from *S. cerevisiae,* the coding region encoding GLO is isolated from *R. norvegicus,* and the coding region encoding AL is isolated from *Z. mobilis.*

Another embodiment is directed to a method of generating ascorbic acid or a salt thereof. The method comprises culturing a recombinant yeast in a medium comprising at least one ascorbic acid precursor, thereby forming ascorbic acid or a salt thereof, and isolating the ascorbic acid or salt thereof from the culture. The recombinant yeast is capable of at least one of (a) converting at least about 25% of the ascorbic acid precursor into ascorbic acid or a salt thereof or (b) producing at least about 20 mg ascorbic acid/ 1 medium in the culture.

In certain embodiments the recombinant yeast is capable of converting at least about 25% of the ascorbic acid in the medium in which the yeast is cultured to ascorbic acid. More preferably, the cultured recombinant yeast is capable of converting at least about 35% of at least one ascorbic acid precursor in the culture medium to ascorbic acid, and most preferably the yeast is capable or converting at least about 40% of the ascorbic acid precursor to ascorbic acid.

Preferably the ascorbic acid is accumulated in the medium at a final concentration of at least about 20 mg ascorbic acid/l culture medium, more preferably at a final concentration of at least about 40 mg ascorbic acid/l culture medium, and most preferably at a final concentration of at least about 70 mg ascorbic acid/l culture medium.

The recombinant yeast is preferably functionally transformed with at least one coding region encoding a protein having an enzyme activity selected from the group consisting of L-galactose dehydrogenase (LGDH), L-galactono-1,4-lactone dehydrogenase (AGD), D-arabinose dehydrogenase (ARA), D-arabinono-1,4-lactone oxidase (ALO), L-gulono-1,4-lactone oxidase (GLO), and aldonolactonase (AL).

In certain embodiments, the recombinant yeast is functionally transformed with a coding region encoding a second enzyme other than the first enzyme, wherein the second enzyme is selected from LGDH, AGD, ARA, ALO, GLO, and AL.

Thus in a preferred embodiment the yeast is functionally transformed with a coding region encoding ALO enzyme. In another embodiment, the yeast is functionally transformed with a coding region encoding LGDH enzyme. In yet another embodiment, the yeast is functionally transformed with a coding region encoding AGD enzyme. In still another embodiment, the yeast is functionally transformed with a coding region encoding GLO enzyme, or the yeast is functionally transformed with a coding region encoding AL enzyme. Alternatively, the yeast is functionally transformed with a coding region encoding ARA enzyme.

In a preferred embodiment, the yeast is functionally transformed with the coding region encoding a protein having D-arabinono-1,4-lactone oxidase (ALO) activity and further functionally transformed with a coding region encoding a protein having L-galactose dehydrogenase (LGDH) activity.

In yet another preferred embodiment, the yeast is functionally transformed with the coding region encoding a protein having D-arabinono-1,4-lactone oxidase (ALO) activity and the coding region encoding a protein having D-arabinose dehydrogenase (ARA) activity.

In yet another preferred embodiment, the yeast is functionally transformed with the coding region encoding a protein having L-galactono-1,4-lactone dehydrogenase (AGD) activity and the coding region encoding a protein having D-arabinose dehydrogenase (ARA) activity. The protein having AGD activity can comprise a signaling peptide in certain embodiments, but does not comprise one in other embodiments.

In yet another preferred embodiment, the yeast is functionally transformed with the coding region encoding a protein having L-galactono-1,4-lactone dehydrogenase (AGD) activity and the coding region encoding a protein having L-galactose dehydrogenase (LGDH) activity. The protein having AGD activity can comprise a signaling peptide in certain embodiments, but does not comprise one in other embodiments.

In another preferred embodiment, the yeast is functionally transformed with the coding region encoding a protein having L-gulono-1,4-lactone oxidase (GLO) activity and the coding region encoding a protein having aldonolactonase (AL) activity.

In a more preferred embodiment, the recombinant yeast further comprises at least one coding region encoding an enzyme associated with the conversion of a carbon source to L-galactose. The enzyme is preferably selected from the group consisting of hexokinase, glucose-6-phosphate isomerase, mannose-6-phosphate isomerase, phosphomannomutase, mannose-1-phosphate guanylyltransferase, GDP-mannose-3,5-epimerase, GDP-L-galactose hydrolase, and L-galactose phosphate phosphatase

In a more preferred embodiment, the recombinant yeast further comprises at least one coding region encoding an enzyme associated with the conversion of a carbon source to L-gulonic acid. The enzyme is preferably selected from the group consisting of hexokinase, phosphoglucomutase, UTP-glucose-1-phosphate uridylyltransferase, UDP-D-glucose dehydrogenase, glucuronate-1-phosphate uridylyltransferase, D-glucurono kinase and D-glucuronate reductase.

In another preferred embodiment, the recombinant yeast further comprises at least one coding region encoding an enzyme associated with the conversion of a carbon source to L-gulono-1,4-lactone. The enzyme is preferably selected from the group consisting of hexokinase, phosphoglucomutase, UTP-glucose-1-phosphate uridylyltransferase, UDP-D-glucose dehydrogenase, glucuronate-1-phosphate uridylyltransferase, D-glucurono kinase, uronolactonase, D-glucuronate reductase and glucurono lactone reductase.

In another more preferred embodiment, the amino acid sequence of the LGDH enzyme or a protein having LGDH activity has at least about 70%, more preferably about 80%, and most preferably about 90% similarity with SEQ ID NO: 11; the amino acid sequence of the AGD enzyme or a protein having AGD activity has at least about 70%, more preferably about 80%, and most preferably about 90% similarity with SEQ ID NO: or SEQ ID NO:3; the amino acid sequence of the ARA enzyme or a protein having ARA activity has at least about 70%, more preferably about 80%, and most preferably about 90% similarity with SEQ ID NO:20; the amino acid sequence of the ALO enzyme or a protein having ALO activity has at least about 70%, more preferably about 80%, and most preferably about 90% similarity with SEQ ID NO:5 or SEQ ID NO:7; the amino acid sequence of the GLO enzyme or a protein having GLO activity has at least about 70%, more preferably about 80%, and most preferably about 90% similarity with SEQ ID NO:9; the AL enzyme or a protein having AL activity has at least about 70%, more preferably about 80%, and most preferably about 90% similarity with SEQ ID NO:29; wherein "similarity" is determined by a sequence alignment performed using the CLUSTAL program.

In another more preferred embodiment, the amino acid sequence of the LGDH enzyme or a protein having LGDH activity has at least about 70%, more preferably about 80%, and most preferably about 90% identity with SEQ ID NO:11 ; the amino acid sequence of the AGD enzyme or a protein having AGD activity has at least about 70%, more preferably about 80%, and most preferably about 90% identity with SEQ ID NO:1 or SEQ ID NO:3; the amino acid sequence of the ARA enzyme or a protein having ARA activity has at least about 70%, more preferably about 80%, and most preferably about 90% identity with SEQ ID NO:20; the amino acid sequence of the ALO enzyme or a protein having ALO activity has at least about 70%, more preferably about 80%, and most preferably about 90% identity with SEQ ID NO:5 or SEQ ID NO:7; the amino acid sequence of the GLO enzyme or a protein having GLO activity has at least about 70%, more preferably about 80%, and most preferably about 90% identity with SEQ ID NO:9; the AL enzyme or a protein having AL activity has at least about 70%, more preferably about 80%, and most preferably about 90% similarity with SEQ ID NO:29; wherein "identity" is determined by a sequence alignment performed using the CLUSTAL program.

In another more preferred embodiment, the coding region encoding the LGDH enzyme or a protein having LGDH activity has at least about 70%, more preferably about 80%, and most preferably about 90% identity with SEQ ID NO 12; the coding region encoding the AGD enzyme or a protein having AGD activity has at least about 70%, more preferably about 80%, and most preferably about 90% identity with SEQ ID NO 2 or nucleotides 56 through 1858 of SEQ ID NO 4; the coding region encoding the ARA enzyme or a protein having ARA activity has at least about 70%, more preferably about 80%, and most preferably about 90% identity with nucleotides 285 through 1319 of SEQ ID NO 21; the coding region encoding the ALO enzyme or a protein having ALO activity has at least about 70%, more preferably about 80%, and most preferably about 90% identity with SEQ ID NO 6 or nucleotides 4 through 1584 of SEQ ID NO 8; the coding region encoding the GLO enzyme or a protein having GLO activity has at least about 70%, more preferably about 80%, and most preferably about 90% identity with nucleotides 24 through 1346 of SEQ ID NO 10; and the coding region encoding the AL enzyme or a protein having AL activity has at least about 70%, more preferably about 80%, and most preferably about 90% identity with SEQ ID NO:30; wherein "identity" is determined by a sequence alignment performed using the CLUSTAL program.

The present invention also relates to polypeptides which are encoded by nucleic acid sequences which are capable of hybridizing under standard conditions with an oligonucleotide probe which hybridizes under the same conditions with the nucleic acid sequence set forth in SEQ ID NO:12, SEQ ID NO:2, nucleotides 56 through 1858 of SEQ ID NO:4, nucleotides 285 through 1319 of SEQ ID NO:21, SEQ ID NO:6, nucleotides 4 through 1584 of SEQ ID.NO:8, nucleotides 24 through 1346 of SEQ ID NO:10; and SEQ ID NO:30, as well as a complementary strand thereof or a subsequence thereof (J. Sambrook, E. F. Fritsch, and T. Maniatus, 1989, Molecular Cloning, A Laboratory Manual, 2nd edition, Cold Spring Harbor, New York). The oligonucleotide probe can consist of the coding region itself or a subsequence thereof. Hybridization indicates that an analogous nucleic acid sequence exists in both the sequence being probed and in the nucleotide sequence shown in SEQ ID NO: 12, SEQ ID NO:2, nucleotides 56 through 1858.of SEQ ID NO:4, nucleotides 285 through 1319 of SEQ ID NO:21, SEQ ID NO:6, nucleotides 4 through 1584 of SEQ ID NO:8, nucleotides 24 through 1346 of SEQ ID NO:10; and SEQ ID NO:30, or a subsequence thereof.

Thus, in another preferred embodiment, the recombinant yeast is functionally transformed with a coding region that is selected from a coding region encoding a protein having LGDH activity that hybridizes to a complementary strand of the polynucleotide set forth in SEQ ID NO:12; a coding region encoding a protein having AGD activity that hybridizes to a complementary strand of the polynucleotide set forth in SEQ ID NO:2 or nucleotides 56 through 1858 of SEQ ID NO:4; a coding region encoding a protein having ARA activity that hybridizes to a complementary strand of the polynucleotide set forth in nucleotides 285 through 1319 of SEQ ID NO:21; a coding region encoding a protein having ALO activity that hybridizes to a complementary strand of the polynucleotide set forth in SEQ ID NO:6 or nucleotides 4 through 1584 of SEQ ID NO:8; a coding region encoding a protein having GLO activity that hybridizes to a complementary strand of the polynucleotide set forth in nucleotides 24 through 1346 of SEQ ID NO:10; and a coding region encoding a protein having AL activity that hybridizes to a complementary strand of the polynucleotide set forth in SEQ ID NO:30; and wherein the coding region hybridizes to the complementary strand of the polynucleotide under stringent hybridization conditions. Stringent hybridization conditions can be of medium or high stringency. Under medium stringency conditions, for example, prehybridization and hybridization occur at 42°C in 5X SSPE, 0.3% SDS, 200 µg/ml sheared and denatured salmon sperm DNA, and 35% formamide, following standard Southern blotting procedures. Under high stringency conditions, for example, prehybridization and hybridization occur at 42°C in 5X SSPE, 0.3% SDS, 200 µg/ml sheared and denatured salmon sperm DNA, and 50% formamide.

The nucleic acid sequences set forth in SEQ ID NO:12, SEQ ID NO:2, nucleotides 56 through 1858 of SEQ ID NO:4, nucleotides 285 through 1319 of SEQ ID NO:21, SEQ ID NO:6, nucleotides 4 through 1584 of SEQ ID NO:8, nucleotides 24 through 1346 of SEQ ID NO:10; and SEQ ID NO:30, or subsequences thereof may be used to identify and clone DNA encoding homologous proteins from other strains of different genera or species that can be used to produce ascorbic acid according to methods well known in the art. Thus, a genomic or cDNA library prepared from such other organisms may be screened for DNA which hybridizes with SEQ ID NO:12, SEQ ID NO:2, nucleotides 56 through 1858 of SEQ ID NO:4, nucleotides 285 through 1319 of SEQ ID NO:21, SEQ ID NO:6, nucleotides 4 through 1584 of SEQ ID NO:8, nucleotides 24 through 1346 of SEQ ID NO:10; and SEQ ID NO:30, or subsequences thereof.

Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify clones or DNA which are homologous with SEQ ID NO:12, SEQ ID NO:2, nucleotides 56 through 1858 of SEQ ID NO:4, nucleotides 285 through 1319 of SEQ ID NO:21, SEQ ID NO:6, nucleotides 4 through 1584 of SEQ ID NO:8, nucleotides 24 through 1346 of SEQ ID NO:10; and SEQ ID NO:30, or subsequences thereof, the immobilized DNA is allowed to hybridize with a probe that hybridizes to one of these sequences. Following hybridization, the carrier material is washed. For example, a Southern blot that has had a probe hybridized to DNA immobilized on it by washing it three times for 30 minutes each using 0.2XSSC, 0.1% SDS at 40°C, more preferably not higher than 45°C, more preferably not higher than 50°C, more preferably not higher than 55°C, even more preferably not higher than 60°C, especially not higher than 65°C. Molecules to which the probe hybridizes under these conditions are detected using methods known in the art. These molecules can then be cloned and functionally transformed to determine that they have the desired enzymatic activity, and to determine the nucleotide sequence identity with SEQ ID NO:12, SEQ ID NO:2, nucleotides 56 through 1858 of SEQ ID NO:4, nucleotides 285 through 1319 of SEQ ID NO:21, SEQ ID NO:6, nucleotides 4 through 1584 of SEQ ID NO:8, nucleotides 24 through 1346 of SEQ ID NO:10; and SEQ ID NO:30.

In another preferred embodiment, the recombinant yeast is functionally transformed with a coding region that is selected from a coding region encoding a protein having LGDH activity and immunological properties of an enzyme having SEQ. ID 11; enzymes having AGD activity and immunological properties of an enzyme having SEQ ID NO:1 or SEQ ID NO:3; enzymes having ARA activity and immunological properties of an enzyme having SEQ ID NO:20; enzymes having ALO activity and immunological properties of an enzyme having SEQ ID NO:5 or SEQ ID NO:7; enzymes having GLO activity and immunological properties of an enzyme having SEQ ID NO:9; and enzymes having AL activity and immunological properties of an enzyme having SEQ. ID NO:29.

Thus, the present invention also relates to polypeptides having (1) LGDH, AGD, ARA, ALO, GLO or AL activity and (2) immunochemical identity or partial immunochemical identity to the polypeptides having amino acid sequences (a) SEQ. ID 11, SEQ ID NO:1, SEQ ID NO:3, SEQ ID NO:20, SEQ ID NO:5, SEQ ID NO:7, SEQ ID NO:9, or SEQ. ID NO:29 and (b) similar enzyme activity. A polypeptide having immunological properties of an enzyme having a particular SEQ ID means that an antiserum containing antibodies against the antigens of the native polypeptide having the particular SEQ ID number reacts with the antigens of the other protein having similar enzyme activity but a different amino acid sequence in an identical fashion such as total fusion of precipitates, identical precipitate morphology, and/or identical electrophoretic mobility using a specific immunochemical technique. A further explanation of immunochemical identity is described by Axelsen, Bock, and Krøll, In N. H. Axelsen, J. Krøll, and B. Weeks, editors, A Manual of Quantitative Immunoelectrophoresis, Blackwell Scientific Publications, 1973, Chapter 10.

Partial immunochemical identity means that an antiserum containing antibodies against the antigens of the native enzyme (e.g., enzymes having amino acid sequences of SEQ. ID 11, SEQ ID NO:1, SEQ ID NO:3, SEQ ID NO:20, SEQ ID NO:5, SEQ ID NO:7, SEQ ID NO:9, and SEQ. ID NO:29) reacts with the antigens of a polypeptide having similar enzyme activity to the native enzyme in a partially identical fashion such as partial fusion of precipitates, partially identical precipitate morphology, and/or partially identical electrophoretic mobility using a specific immunochemical technique. A further explanation of partial immunochemical identity is described by Bock and Axelsen, In N. H. Axelsen, J. Krøll, and B. Weeks, editors, A Manual of Quantitative Immunoelectrophoresis, Blackwell Scientific Publications, 1973, Chapter 11.

The immunological properties are determined by immunological cross-reaction identity tests by the well-known Ouchterlony double immunodiffusion procedure. Specifically, an antiserum against the polypeptide of the invention is raised by immunizing rabbits (or other rodents according to the procedure described by Harboe and Ingild, In N. H. Axelsen, J. Krøll, and B. Weeks, editors, A Manual of Quantitative Immunoelectrophoresis, Blackwell Scientific Publications, 1973, Chapter 23, or Johnstone and Thorpe, Immunochemistry in Practice, Blackwell Scientific Publications, 1982 (more specifically pages 27-31)). Monoclonal antibodies may be prepared, e.g., according to the methods of E. Harlow and D. Lane, editors, 1988, Antibodies, A Laboratory Manual, Cold Spring Harbor Press, Cold Spring Harbor, New York. Purified immunoglobulins may be obtained from the antiserum, e.g., by ammonium sulfate precipitation, followed by dialysis and ion exchange chromatography (e.g., DEAE-Sephadex). Homologous polypeptides and polypeptides having identical or partially identical immunological properties may be obtained from microorganisms of any genus, preferably from a plant, animal or fungal source.

In another preferred embodiment, wherein the enzyme is ARA, the enzyme comprises motif I and motif II of the aldo-keto reductase (AKR) superfamily, specifically the amino acid sequences GXRXXDXAXXXXXEXXXG (SEQ ID NO:13) and GXXN (SEQ ID NO:26), respectively (Kim S.T. et al. 1998, BBA, 1429, 29-39).

Preferably, a coding region encoding a desired enzyme is incorporated into the yeast in such a manner that the desired enzyme is produced in the yeast and is substantially functional. Such a yeast may be referred to herein as being "functionally transformed."

Once the coding region has been isolated, it can be prepared for transformation into and expression in the yeast useful in the present invention. At minimum, this involves the insertion of the coding region into a vector and operable linkage to a promoter found on the vector and active in the target organism (i.e., in the present invention, a yeast). Any vector (integrative, chromosomal or episomal) can be used.

Any promoter active in the target host (homologous or heterologous, constitutive, inducible or repressible) can be used. Such insertion often involves the use of restriction, endonucleases to "open up" the vector at a desired point where operable linkage to the promoter is possible, followed by ligation of the coding region into the desired point. If desired, before insertion into the vector, the coding region can be prepared for use in the target organism. This can involve altering the codons used in the coding region to more fully match the codon use of the target organism; changing sequences in the coding region that could impair the transcription or translation of the coding region or the stability of an mRNA transcript of the coding region; or adding or removing portions encoding signaling peptides (regions of the protein encoded by the coding region that direct the protein to specific locations (e.g. an organelle, the membrane of the cell or an organelle, or extracellular secretion)), among other possible preparations known in the art.

In one embodiment of the present invention, the L-galactono-1,4-lactone dehydrogenase protein (AGD) comprises a signaling peptide and the coding region encoding the L-galactono-1,4-lactone dehydrogenase also encodes the signaling peptide. In another embodiment of the present invention, the L-galactono-1,4-lactone dehydrogenase protein (AGD) does not comprise a signaling peptide and the coding region encoding the L-galactono-1,4-lactone dehydrogenase also does not encode the signaling peptide. Specifically, the AGD sequence given in SEQ ID NO:1 comprises a signaling peptide of amino acids 1-100, and the AGD sequence given in SEQ ID NO:2 comprises a signaling peptide of amino acids 1-90. As one of skill in the art will recognize, deletion of a nucleic acid sequence encoding a signaling peptide from a longer nucleic acid sequence encoding a desired enzyme may require the addition of an in-frame ATG codon to allow for proper initiation of translation of the desired enzyme.

Regardless whether the coding region is modified, when the coding region is inserted into the vector, it is operably linked to a promoter active in the yeast. A promoter, as is known, is a DNA sequence that can direct the transcription of a nearby coding region. As already described, the promoter can be constitutive, inducible or repressible. Inducible promoters can be induced by the addition to the medium of an appropriate inducer molecule or by an appropriate change of the chemical or physical growth environment (such as the temperature, pH values), which will be determined by the identity of the promoter. Repressible promoters can be repressed by the addition to the medium of an appropriate repressor molecule or by an appropriate change of the chemical or physical growth environment (such as the temperature, pH values), which will be determined by the identity of the promoter. Constitutive promoters are preferred, as the use of an inducer or repressor molecule or a change of the chemical or physical growth environment is not required. A preferred constitutive promoter is the *S*. *cerevisiae* triosephosphateisomerase (TPI) promoter.

The vector comprising the coding region operably linked to the promoter can be a plasmid, a cosmid, or a yeast artificial chromosome, among others known in the art to be appropriate for use in yeast genera. In addition to the coding region operably linked to the promoter, the vector can also comprise other genetic elements. For example, if the vector is not expected to integrate into the yeast genome, the vector desirably comprises an origin of replication, which allows the vector to be passed on to progeny cells of a yeast comprising the vector. If integration of the vector into the yeast genome is desired, the vector can comprise sequences homologous to sequences found in the yeast genome, and can also comprise coding regions that can facilitate integration. To determine which yeast cells are transformed, the vector preferably comprises a selectable marker or screenable marker which imparts a phenotype to the yeast that distinguishes it from untransformed yeast, e.g. it survives on a medium comprising an antibiotic fatal to untransformed yeast or it metabolizes a component of the medium into a product that the untransformed yeast does not, among other phenotypes. In addition, the vector may comprise other genetic elements, such as restriction endonuclease sites and others typically found in vectors.

After the vector is prepared, with the coding region operably linked to the promoter, the yeast is transformed with the vector (i.e. the vector is introduced into at least one of the cells of a yeast population). Techniques for yeast transformation are well established, and include electroporation, microprojectile bombardment, and the LiAc/ssDNA/PEG method, among others. Yeast cells, which are transformed, can then be detected by the use of a screenable or selectable marker on the vector. It should be noted that the phrase "transformed yeast" has essentially the same meaning as "recombinant yeast," as defined above. The transformed yeast can be one that received the vector in a transformation technique, or can be a progeny of such a yeast.

Certain embodiments are directed to a recombinant yeast that is functionally transformed with at least one coding region encoding a protein having an enzyme activity selected from the group consisting of L-galactose dehydrogenase (LGDH), L-galactono-1,4-lactone dehydrogenase (AGD), D-arabinose dehydrogenase (ARA), D-arabinono-1,4-lactone oxidase (ALO), L-gulono-1,4-lactone oxidase (GLO), and aldonolactonase (AL), as described above.

After a recombinant yeast has been obtained, the yeast is cultured in a medium. The medium is as described above.

A preferred medium comprises glucose, YNB, and L-galactono-1,4-lactone. Preferred recombinant yeasts which can be cultured in this medium include a *S*. *cerevisiae* strain (preferably GRF18U) bearing a *S*. *cerevisiae* TPI promoter operably linked to a coding region encoding *A*. *thaliana* L-galactono-1,4-lactone dehydrogenase (AGD); and a *S*. *cerevisiae* strain (preferably GRF1.8U) bearing a *S*. *cerevisiae* TPI promoter operably linked to a coding region encoding *S. cerevisiae* D-arabinono-1,4-lactone oxidase (ALO). Other preferred recombinant yeast which can be cultured in this medium include a *Z. bailii* strain (preferably ATCC 36947) bearing a *S. cerevisiae* TPI promoter operably linked to a coding region encoding *S. cerevisiae* D-arabinono-1,4-lactone oxidase (ALO); and a *Z. bailii* strain (preferably ATCC 36947) bearing a *S. cerevisiae* TPI promoter operably linked to a coding region encoding *S*. *cerevisiae* L-galactono-1,4-lactone dehydrogenase (AGD). Another preferred medium comprises glucose, YNB and L-gulono-1,4-lactone. One particularly preferred recombinant yeast which can be cultured in this medium is a *S*. *cerevisiae* strain (preferably GRF18U) bearing a *S*. *cerevisiae* TPI promoter operably linked to a coding region encoding *R. norvegicus* L-gulono-1,4-lactone oxidase (RGLO). One other particularly preferred recombinant yeast which can be cultured in this medium is a *Z. bailii* strain (preferably ATCC 36947 or 60483) bearing a *S*. *cerevisiae* TPI promoter operably linked to a coding region encoding *R. norvegicus* L-gulono-1,4-lactone oxidase (RGLO).

Another preferred medium comprises glucose, YNB and L-galactose. One particularly preferred transformed yeast which can be cultured in this medium is a *S. cerevisiae* strain (preferably GRF18U) or a *Z. bailii* strain (preferably ATCC 36947) bearing (i) a *S. cerevisiae* TPI promoter operably linked to a coding region encoding *A. thaliana* L-galactono-1,4-lactone dehydrogenase (AGD) and (ii) a TPI promoter operably linked to a coding region encoding *A. thaliana* L-galactose dehydrogenase (LGDH). A second particularly preferred transformed yeast which can be cultured in this medium is a *S. cerevisiae* strain (preferably GRF18U) or a *Z. bailii* strain (preferably ATCC 36947) comprising (i) a TPI promoter operably linked to a coding region encoding *S. cerevisiae* D-arabinono-1,4-lactone oxidase (ALO) and (ii) a TPI promoter operably linked to a coding region encoding *A. thaliana* L-galactose dehydrogenase (LGDH). A third particularly preferred transformed yeast which can be cultured in this medium is a *S*. *cerevisiae* strain (preferably GRF18U) or a *Z*. *bailii* strain (preferably ATCC 36947) comprising (i) a TPI promoter operably linked to a coding region encoding *S*. *cerevisiae* D-arabinono-1,4-lactone oxidase (ALO) and (ii) a TPI promoter operably linked to a coding region encoding *S. cerevisiae* D-arabinose dehydrogenase (ARA). A fourth particularly preferred yeast which can be cultured in this medium is a *S. cerevisiae* strain (preferably GRF18U) or a *Z. bailii* strain (preferably ATCC 36947) comprising (i) a TPI promoter operably linked to a coding region encoding *A. thaliana* L-galactono-1,4-lactone dehydrogenase (AGD) and (ii) a TPI promoter operably linked to a coding region encoding *S. cerevisiae* D-arabinose dehydrogenase (ARA). Another preferred recombinant yeast which can be culture in this medium is a *S. cerevisiae* strain (preferably GRP18U) or a *Z. bailii* strain (preferably ATCC 36947) bearing a *S*. *cerevisiae* TPI promoter operably linked to a coding region encoding *S. cerevisiae* D-arabinose dehydrogenase (ARA). Still another preferred recombinant yeast which can be cultured in this medium is a *S. cerevisiae* strain (preferably GRF18U) or a *Z*. *bailii* strain (preferably ATCC 36947) bearing a *S. cerevisiae* TPI promoter operably linked to a coding region encoding *A. thaliana* L-galactose dehydrogenase (LGDH).

Another preferred medium comprises glucose, YNB and L-gulonic acid. One particularly preferred recombinant yeast which can be cultured in this medium is a *S. cerevisiae* strain (preferably GRF18U) or a *Z. bailii* strain (preferably ATCC 36947) bearing a *S*. *cerevisiae* TPI promoter operably linked to a coding region encoding *Z. mobilis* aldonolactonase (AL). Another preferred recombinant yeast, which can be cultured in this medium is a *S. cerevisiae* strain (preferably GRF18U) or a *Z. bailii* strain (preferably ATCC 36947) bearing (i) a *S*. *cerevisiae* TPI promoter operably linked to a coding region encoding *R. norvegicus* L-gulono-1,4-lactone oxidase (RGLO) and (ii) a TPI promoter operably linked to a coding region encoding *Z. mobilis* aldonolactonase (AL).

As described for non-recombinant yeast, above, during the course of the fermentation, the ascorbic acid precursor is converted, through one or more steps, into L-ascorbic acid.

While the non-recombinant yeast cells (described above) incubated in similar media typically do not accumulate ascorbic acid above background levels in the medium, surprisingly, some of the particularly preferred recombinant strains herein described are able to accumulate considerable amounts of L-ascorbic acid above background levels. A first exception relates to a yeast transformed with only LGDH, which does not accumulate L-ascorbic acid above background levels. A second exception relates to a yeast transformed with RGLO and/or AL, which does not accumulate L-ascorbic acid above background levels. Therefore, in a preferred embodiment, the recombinant yeast accumulates L-ascorbic acid in the medium above background levels.

Isolation of the ascorbic acid from the media is as described above. Yields of ascorbic acid of greater than about 25% and 35% have been observed, as will be described in the Examples below. Therefore, in a further preferred embodiment, the recombinant yeast produce ascorbic acid with a yield higher than 25% of the precursor. The term "yield" refers to the amount of ascorbic acid (molar as well as weight/volume) produced divided by the amount of precursor consumed (molar as well as weight/volume) multiplied by 100.

### The following definitions are provided in order to aid those skilled in the art in understanding the detailed description of the present invention.

The term "accumulation of ascorbic acid above background levels" refers to the accumulation of ascorbic acid above the undetectable levels as determined using the procedures described herein.

"Ascorbic acid" as well as "ascorbate" as used herein, refers to vitamin C, L-ascorbic acid and salts or ions thereof.

"Ascorbic acid precursor" is a compound that can be converted by a yeast of the present invention, either directly or through one or more intermediates, into L-ascorbic acid or a salt thereof.

"Amplification" refers to increasing the number of copies of a desired nucleic acid molecule or to increase the activity of an enzyme, by whatsoever means.

"Carbon source" refers to an organic compound used by yeast in culture as a source of carbon to produce new biomass and in cellular metabolism.

"Codon" refers to a sequence of three nucleotides that specify a particular amino acid. "DNA ligase" refers to an enzyme that covalently joins two pieces of double-stranded DNA.

"Electroporation" refers to a method of introducing foreign DNA into cells that uses a brief, high voltage DC charge to permeabilize the host cells, causing them to take up extra-chromosomal DNA.

"Endonuclease" refers to an enzyme that hydrolyzes double stranded DNA at internal locations.

Enzyme 1.1.3.37, D-arabinono-1,4-lactone oxidase, refers to a protein that catalyzes the conversion of D-arabinono-1,4-lactone + O₂ to D-erythroascorbate + H₂O₂. The same enzyme due to broadness of substrate range catalyses the conversion of L-galactono-1,4-lactone + O₂ to L-ascorbic acid + H₂O₂. Erroneously the same enzyme is referred to as L-galactono-1,4-lactone oxidase (enzyme 1.1.3.24) (see Huh, W.K. et al, 1998, Mol. Microbiol. 30, 895-903)(Nishikimi, M., et al., 1978, Arch. Biochem. Biophys. 191, 479-486)(Bleeg, H.S. and Christensen, F., 1982, Eur. J. Biochem. 127, 391-96)..

Enzyme 1.3.2.3, L-galactono-1,4-lactone dehydrogenase, refers to a protein that catalyzes the conversion of L-galactono-1,4-lactone + 2 ferricytochrome C to L-ascorbic acid + 2 ferrocytochrome C.

Enzyme 1.1.3.8, L-gulono-1,4-lactone oxidase, refers to a protein that catalyzes the oxidation of L-gulono-1,4-lactone to L-xylo-hexulonolactone, which spontaneously isomerizes to L-ascorbic acid.

Other enzymes of interest, and their classification numbers, are as follows:

| | |
|---|---|
| Hexokinase | 2.7.1.1 |
| Glucose-6-P isomerase | 5.3.1.9 |
| Mannose-6-P isomerase | 5.3.1.8 |
| Phosphomannomutase | 5.4.2.8 |
| Mannose-1-P guanylyltransferase | 2.7.7.22 |
| GDP-Mannose 3,5-epimerase | 5.1.3.18 |
| Sugar phosphatase | 3.1.3.23 |
| L-Galactose-dehydrogenase | *) |
| L-Galactono-1,4-lactone dehydrogenase | 1.3.2.3 |
| D-Mannose kinase | 2.7.1.1 |
| Phosphoglucomutase | 5.4.2.2 |
| UTP-Glucose-1-P uridylyl transferase | 2.7.7.9 |
| UDP-D-Glucose dehydrogenase | 1.1.1.22 |
| UDP-Glucuronate 4-epimerase | 5.1.3.6 |
| Glucuronate-1-P uridylyltransferase | 2.7.7.44 |
| D-Glucuronokinase | 2.7.1.43 |
| D-Glucuronate reductase | 1.1.1.19 |
| Aldonolactonase | 3.1.1.17 |
| L-Gulono-1,4-lactone oxidase | 1.1.3.8 |
| Uronolactonase | 3.1.1.19 |
| Glucuronolactone reductase activity | 1.1.1.20 |
| L-Galactono-1,4-lactone 3-epimerase | *) |
| Galacturonate-1-P uridylyltransferase | *) |
| Galacturonokinase | 2.7.1.44 |
| Hexuronate (D-galacturonate) reductase | *) |
| Myoinositol 1-P synthase | 5.5.1.4 |
| Myoinositol 1-P monophosphatase | 3.1.3.25 |
| Myoinositol oxygenase | 1.13.99.1 |
| D-Galactokinase | 2.7.1.6 |
| UTP-Hexose 1-P uridylyltransferase | 2.7.7.10 |
| UDP-Glucose 4-epimerase | 5.1.3.2 |
| Suc synthase | 2.4.1.13 |
| Fructokinase | 2.7.1.4 |

| | |
|---|---|
| *) Classification number not available in databases. | |

The term "expression" refers to the transcription of a gene to produce the corresponding mRNA and translation of this mRNA to produce the corresponding gene product, i.e., a peptide, polypeptide, or protein.

The phrase "functionally linked" or "operably linked" refers to a promoter or promoter region and a coding or structural sequence in such an orientation and distance that transcription of the coding or structural sequence may be directed by the promoter or promoter region.

The term "gene" refers to chromosomal DNA, plasmid DNA, cDNA, synthetic DNA, or other DNA that encodes a peptide, polypeptide, protein, or RNA molecule, and regions flanking the coding sequence involved in the regulation of expression.

The term "genome" encompasses both the chromosomes and plasmids within a host cell. Encoding DNAs of the present invention introduced into host cells can therefore be either chromosomally integrated or plasmid-localized.

"Heterologous DNA" refers to DNA from a source different than that of the recipient cell.

"Homologous DNA" refers to DNA from the same source as that of the recipient cell.

"Hybridization" refers to the ability of a strand of nucleic acid to join with a complementary strand via base pairing. Hybridization occurs when complementary sequences in the two nucleic acid strands bind to one another.

The term "medium" refers to the chemical environment of the yeast comprising any component required for the growth of the yeast or the recombinant yeast and one or more precursors for the production of ascorbic acid. Components for growth of the yeast and precursors for the production of ascorbic acid may or may be not identical.

"Open reading frame (ORF)" refers to a region of DNA or RNA encoding a peptide, polypeptide, or protein.

"Plasmid" refers to a circular, extra chromosomal, replicatable piece of DNA.

"Polymerase chain reaction (PCR)" refers to an enzymatic technique to create multiple copies of one sequence of nucleic acid. Copies of DNA sequence are prepared by shuttling a DNA polymerase between two amplimers. The basis of this amplification method is multiple cycles of temperature changes to denature, then re-anneal amplimers, followed by extension to synthesize new DNA strands in the region located between the flanking amplimers.

The term "promoter" or "promoter region" refers to a DNA sequence, usually found upstream (5') to a coding sequence, that controls expression of the coding sequence by controlling production of messenger RNA (mRNA) by providing the recognition site for RNA polymerase and/or other factors necessary for start of transcription at the correct site.

A "recombinant cell" or "transformed cell" is a cell that contains a nucleic acid sequence not naturally occurring in the cell or an additional copy or copies of an endogenous nucleic acid sequence, wherein the nucleic acid sequence is introduced into the cell or an ancestor thereof by human action.

The term "recombinant vector" or "recombinant DNA or RNA construct" refers to any agent such as a plasmid, cosmid, virus, autonomously replicating sequence, phage, or linear or circular single-stranded or double-stranded DNA or RNA nucleotide sequence, derived from any source, capable of genomic integration or autonomous replication, comprising a nucleic acid molecule in which one or more sequences have been linked in a functionally operative manner. Such recombinant constructs or vectors are capable of introducing a 5' regulatory sequence or promoter region and a DNA sequence for a selected gene product into a cell in such a manner that the DNA sequence is transcribed into a functional mRNA, which may or may not be translated and therefore expressed.

"Restriction enzyme" refers to an enzyme that recognizes a specific sequence of nucleotides in double stranded DNA and cleaves both strands; also called a restriction endonuclease. Cleavage typically occurs within the restriction site or close to it.

"Selectable marker" refers to a nucleic acid sequence whose expression confers a phenotype facilitating identification of cells containing the nucleic acid sequence. Selectable markers include those, which confer resistance to toxic chemicals (e.g. ampicillin, kanamycin, G418, hygromycin) or complement a nutritional deficiency (e.g. uracil, histidine, leucine).

"Screenable marker" refers to a nucleic acid sequence whose expression imparts a visually distinguishing characteristic (e.g. color changes, fluorescence).

"Transcription" refers to the process of producing an RNA copy from a DNA template.

"Transformation" refers to a process of introducing an exogenous nucleic acid sequence (e.g., a vector, plasmid, or recombinant nucleic acid molecule) into a cell in which that exogenous nucleic acid is incorporated into a chromosome or is capable of autonomous replication. A cell that has undergone transformation, or a descendant of such a cell, is "transformed" or "recombinant." If the exogenous nucleic acid comprises a coding region encoding a desired protein, and the desired protein is produced in the transformed yeast and is substantially functional, such a transformed yeast is "functionally transformed."

"Translation" refers to the production of protein from messenger RNA.

The term "yield" refers to the amount of ascorbic acid produced (molar or weight/volume) divided by the amount of precursor consumed (molar or weight/volume) multiplied by 100.

"Unit" of enzyme refers to the enzymatic activity and indicates the amount of micromoles of substrate converted per mg of total cell proteins per minute.

"Vector" refers to a DNA or RNA molecule (such as a plasmid, cosmid, bacteriophage, yeast artificial chromosome, or virus, among others) that carries nucleic acid sequences into a host cell. The vector or a portion of it can be inserted into the genome of the host cell.

### List of abbreviations:

- Asc: L-ascorbic acid (vitamin C) or a salt thereof
- AGD: L-galactono-1,4-lactone dehydrogenase
- AL: aldonolactonase
- ALO: D-arabinono-1,4-lactone oxidase
- ARA: D-arabinose dehydrogenase
- Gal: L-galactono-1,4-lactone
- Gul: L-gulono-1,4-lactone
- LGDH: L-galactose dehydrogenase
- GLO: L-gulono-1,4-lactone oxidase
- RGLO: L-gulono-1,4-lactone oxidase isolated from *R. norvegicus*
- TCA: trichloro acetic acid
- TPI: triosephosphateisomerase
- OD⁶⁶⁰: optical density at 660 nm

It should be noted that the abbreviations for the enzyme activities refer to the respective activities and not to a specific enzyme.

### EXAMPLES

The following examples are included to demonstrate preferred embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Materials and Methods

### 1. Determination of ascorbic acid

Ascorbic acid was determined spectrophotometrically following a method after Sullivan et al. (1955, Assoc. Off. Agr. Chem., 38, 514-518). 135 µl of sample were mixed in a cuvette with 40 µl of H₃PO₄ (85%). Then 675 µl ,-Bipyridyl (0.5%) and 135 µl FeCl₃ (1%) were added. After 10 min the absorbance at 525nm was measured. The identity of the ascorbic acid was confirmed by HPLC (Tracer Extrasil Column C8, 5 µM, 15 x 0.46 cm, Teknokroma, S. Coop. C. Ltda. # TR-016077; Eluent: 5 mM cetyltrimethylammonium bromide, 50 mM KH₂PO₄ in 95/5 H₂O/Acetonitrile; Flow rate: 1 ml min⁻¹, Detection UV @ 254 nm) with pure L-ascorbic acid (Aldrich, A9,290-2) as standard.

### 2. Determination of protein concentration

Protein concentrations were determined following Lowry's method (Lowry O.H. et al., 1951, J. Biol. Chem. 193, 265-275), using the Bio-Rad DC Protein Assay Kit II (Cat. Nr. 500-0112) with BSA as standard.

### 3. Amplification of specific gene sequences

To amplify specific gene sequences, PfuTurbo DNA polymerase (Stratagene #600252) was used on a GeneAmp PCR System 9700 (PE Appl. Biosystems, Inc.). Standard conditions used were: 400 µM dNTP, 0.5 µM primers, 0.5 mM MgCl₂ (in addition to the buffer), and 3.75 U Pfu per 100 µl reaction.

The sequences of the genes used have been publicly reported via Genbank, as follows

| Gene | Genbank accession no(s). | Nucleotide Sequence Including Coding Region SEQ ID NO: | Amino Acid Sequence SEQ ID NO: |
|---|---|---|---|
| LGDH | AL031394 (Gene no. T16L.160) | 12 | 11 |
| AGD | AL049658 (Gene no. T17F15.200) | 2 | 1 |
| AGD homolog from *Brassica* | Z97060 | 4 | 3 |
| AL | X67189; S53050 | 30 | 29 |
| ALO | U40390, AB009401 | 6,8 | 5,7 |
| RGLO | J03536 | 10 | 9 |
| ARA | Y13134, Z36018 (ORF YBR149w) | 21 | 20 |

The following program was used for amplification of AGD:

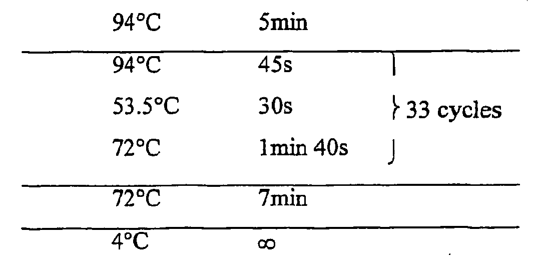

The following program was used for amplification of AL:

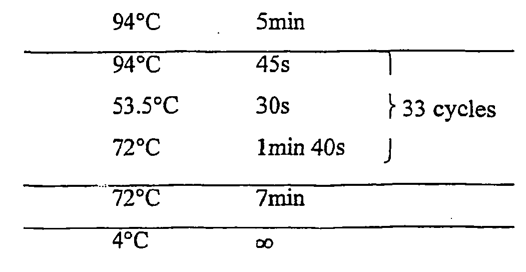

The following program was used for amplification of ALO:

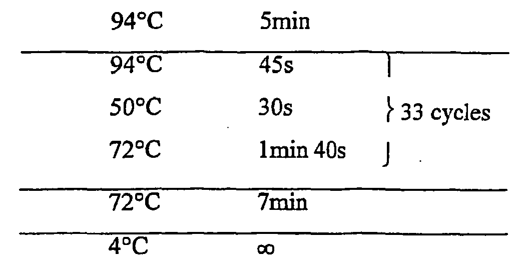

The following program was used for amplification of ARA:

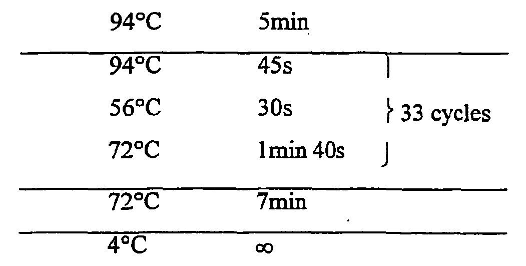

The following program was used for amplification of LGDH:

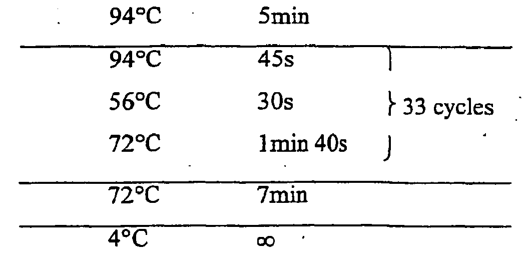

The following program was used for amplification of RGLO:

| | | |
|---|---|---|
| 94°C | 30s | |
| 94°C | 5s | |
| 72°C | 4min | 33 cycles |
| 72°C | 5min | |
| 4°C | ∞ | |

Template DNA for AGD and LGDH: 50 ng plasmid cDNA library pFL61 *Arabidopsis* (ATCC #77500 (Minet M. et al, 1992, Plant J., 2, 417-422)). Template DNA for RGLO: 0.5 ng rat liver marathon-ready cDNA library (Clontech #7471-1). Template DNA for AL: 50 ng of genomic DNA from *Zymomonas mobilis* (ATCC #10988), extracted using a standard method. Template DNA for ALO and ARA: 50 ng genomic DNA from *S. cerevisiae* GRF18U, extracted using a standard method. PCR products were blunt end cloned into the EcoRV site of pSTBlue-1 using the perfectly blunt cloning kit from Novagen Inc. (#70191-4).

| Oligonucleotides used | | Gene amplified |
|---|---|---|
| SEQ ID NO:14: | caagaaggcctaaatgttccgttacgctcc | |
| SEQ ID NO:15: | atgggcccttaagcagtggtggagactggg | AGD |
| | | |
| SEQ ID NO: 16: | tgaggggtcagggtggtttgtttcca | |
| SEQ ID NO:17: | tggaatcatggtccatgggtacaaaggg | RGLO |
| | | |
| SEQ ID NO:18: | tttcaccatatgtctactatcc | |
| SEQ ID NO: 19: | aaggatcctagtcggacaactc | ALO |
| | | |
| SEQ ID NO:22: | atgacgaaaatagagcttcgagc | |
| SEQ ID NO:23: | ttagttctgatggattccacttgg | LGDH |
| | | |
| SEQ ID NO:24: | atgtcttcttcagtagcctcaacc | |
| SEQ ID NO:25: | ttaatactttaaattgtccaagtttggtc | ARA |
| | | |
| SEQ ID NO:27: | atgaccactggtcgtatgtctcg | |
| SEQ ID NO:28: | ttaccagaaaataagacccaagca | AL |

### 4. Plasmid construction

The naming convention used herein is that pSTBlue-1 containing, for example, AGD in sense direction regarding its multiple cloning site (MCS) was designated pSTB AGD-1. In a further example, pSTBlue-1 containing AGD in antisense direction regarding its MCS was designated pSTB AGD-2, and so on.

Standard procedures were employed for all cloning purposes (Sambrook J. et al., Molecular Cloning: A Laboratory Manual, Cold Spring Harbor Laboratory Press).

| Insert derives from | Insert cut with | | Target plasmid | Target plasmid cut with | Name of the resulting expression plasmid |
|---|---|---|---|---|---|
| pSTB AGD-1 | EcoRI | | pYX042 | EcoRI | pL-AGD |
| pSTB LGDH-1 | EcoRI | | pYX022 | EcoRI | pH-LGDH |
| pSTB ALO-1 | EcoRI | | pYX042 | EcoRI | pL-ALO |
| pSTB AL-1 | EcoRI | | pYX022 | EcoRI | pH-AL |
| pSTB ALO-1 | EcoRI | | pZ3 | EcoRI | pZ3-ALO |
| pH-LGDH | AatII/PvuII/blunt | | pAG26 | ApaI/blunt | pAG26TPI-LGDH |
| pSTBARA-2 | SacI blunt | BamHI | pYX022 | EcoRI blunt BamHI | pH-ARA |
| pSTB RGLO-1 | NotI blunt | KpnI blunt | pYX042 | EcoRI blunt | pL-RGLO |
| pSTB RGLO-1 | NotI blunt | KpnI blunt | pZ3 | EcoRI blunt | pZ3-RGLO |

### Expression plasmid construction:

The pYX plasmids are a series of commercially available yeast expression vectors (R&D Systems, Inc.).

### pAG26TPI:

The expression cassette (consisting of TPI promoter, multiple cloning site and terminator) from pYX022 was cut with restriction enzymes AatII/PvuII, blunt ended and cloned into pAG26 (Goldstein A.L. and McCusker J.H., 1999, Yeast 15, 1541-1553) which had been cut with restriction enzyme ApaI and made to have blunt ends.

### pZ3:

The ARS/CEN region was cut from YCplac33 (Gietz R.D. and Sugino A., 1988, Gene 74, 527-534) by cutting it with the restriction enzyme ClaI, making the ends blunt, and cutting with the restriction enzyme SpeI and cloned into pYx022, which had been cut with the restriction enzyme DraIII, treated to make the ends blunt, and cut with the restriction enzyme SpeI. The resulting plasmid was opened with the restriction enzyme KpnI and blunt ended, to receive the G418 resistance cassette, cut from pFA6-KanMX4 (Wach et al., 1994, Yeast 10, 1793-1808) with restriction enzymes SphI/SacI and blunt ended.

### 5. Yeast Cultivation and examination:

Yeast strains used were *S. cerevisiae* GRF18U, W3031B, *Z. bailii* ATCC 60483, *Z. bailii* ATCC 36947, and *K. lactis* PM6-7A (Wésolowski-Louvel, M. et al., 1992, Yeast 8, 711-719). GRF18U has been deposited with the Agricultural Research Service Culture Collection and has the following catalog number NRRL Y-30320. All strains were cultivated in shake flasks in minimal medium (0.67% w/v YNB (Difco Laboratories, Detroit, MI #919-15), 2% w/v glucose, addition of the appropriate amino acids or adenine or uracil, respectively, to 50 mg 1⁻¹) under standard conditions (shaking at 30°C). The initial optical density at 660 nm was about 0.05.

For incubation with L-galactose the cells were grown over night, then 250 mg l⁻¹ of L-galactose were added and the cells were incubated for 24 hr. For incubation with substrates other than L-galactose, the cells were grown in presence of 50 mM or 100 mM of the respective substrates for 72 hr if not stated differently. For the determination of intracellular ascorbic acid, cells were treated as follows: cells were recovered by centrifugation at 4000 rpm for 5 min at 4°C, washed once with cold distilled H₂O, resuspended in about 3 times the pellet volume of cold 10% TCA, vortexed vigorously, kept on ice for about 20 min, and then the supernatant was cleared from the cell debris by centrifugation.

### 6. Yeast transformation:

Transformation of yeast cells was done following the standard LiAc/ss-DNA/PEG method (Agatep, R., et al., 1998). *S. cerevisiae* GRF 18U and various transformed yeast were deposited with NRRL. The table below lists the strains and their NRRL numbers.

| | **Strain** | **NRRL Number** | **Deposit Date** |
|---|---|---|---|
| 1 | *S. cerevisiae* GRF18U | NRRL Y-30320 | July 31, 2000 |
| 2 | *S. cerevisiae* GRF18U [pL-ALO) | NRRL Y-30321 | July 31, 2000 |
| 3 | *S. cerevisiae* GRF18U [pL-AGD] | NRRL Y-30322 | July 31, 2000 |
| 4 | *S. cerevisiae* GRF18U [pH-LGDH] | NRRL Y-30323 | July 31, 2000 |
| 5 | *S. cerevisiae* GRF18U [pH-LGDH] [pL-ALO] | NRRL Y-30324 | July 31, 2000 |
| 6 | *S. cerevisiae* GRF18U [pH-LGDH] [pL-AGD] | NRRL Y-30325 | July 31, 2000 |
| 7 | *S. cerevisiae* GRF18U [pH-ARA] [pL-ALO] | NRRL Y-30326 | July 31, 2000 |
| 8 | *S. cerevisiae* W3031B [pL-ALO] | NRRL Y-30327 | July 31, 2000 |
| 9 | *S. cerevisiae* W3031B [pH-LGDH] [pL-ALO] | NRRL Y-30328 | July 31, 2000 |
| 10 | *S. cerevisiae* W3031B [PH-ARA][pL-ALO] | NRRL Y-30329 | July 31, 2000 |
| 11 | *S. cerevisiae* GRF18U [pL] | NRRL Y-30490 | July 20, 2001 |
| 12 | *S. cerevisiae* GRF18U [pL-RGLO] | NRRL Y-30491 | July 20, 2001 |
| 13 | *S. cerevisiae* GRF18U [pH-AL] [pL] | NRRL Y-30492 | July 20, 2001 |
| 14 | *S. cerevisiae* GRF18U [pH-AL] [pL-RGLO] | NRRL Y-30493 | July 20, 2001 |
| 15 | *Z. bailii* ATCC 36947 [pZ3] | NRRL Y-30494 | July 20, 2001 |
| 16 | *Z. bailii ATCC* 36947 [pZ3-RGLO] | NRRL Y-30495 | July 20, 2001 |
| 17 | *Z. bailii* ATCC 60483 [pZ3] | NRRL Y-30496 | July 20, 2001 |
| 18 | *Z. bailii* ATCC 60483 [pZ3-RGLO] | NRRL Y-30497 | July 20, 2001 |
| 19 | *Z. bailii* ATCC 36947 [pZ3-ALO] | NRRL Y-30498 | July 20, 2001 |
| 20 | *Z. bailii* ATCC 36947 [pAG26TPI] [pZ3-ALO] | NRRL Y-30499 | July 20, 2001 |
| 21 | *Z. bailii* ATCC 36947 [pAG26TPI-LGDH] | NRRL Y-30500 | July 20, 2001 |
| 22 | *Z. bailii* ATCC 36947 [pAG26TPI-LGDH] [pZ3-ALO] | NRRL Y-30501 | July 20, 2001 |

The strains were deposited with the Agricultural Research Service Culture Collection, 1815 North University Street, Peoria, Illinois 61604, USA. This deposit was made under the provisions of the Budapest Treaty on the International Recognition of the Deposit of Microorganisms for the Purposes of Patent Procedure and the regulations thereof (Budapest Treaty). The yeast strains will be made available by the NRRL under the terms of the Budapest Treaty upon issue of a U.S. patent with pertinent claims. Availability of the deposited yeasts is not be construed as a license to practice the invention in contravention of the rights granted under the authority of any government in accordance with its patent laws.

### Experimental Results

### 1. Stability of L-ascorbic acid

To determine the stability of ascorbic acid under culture conditions, we added ascorbic acid to our standard medium (2% glucose, 0.67% YNB) and incubated the solution in shake flasks shaking at 30°C. Figure 2 shows the respective results. In sterile medium, ascorbic acid is rapidly degraded (see panel B), whereas it is completely stable if viable yeast are present (see panel A). This result shows that culturing yeast in a medium is a method of stabilizing ascorbic acid.

Therefore the ascorbic acid potentially produced by yeast is stabilized by the presence of the yeast, making methods of the present invention suitable for the production of ascorbic acid.

### 2. Ascorbic acid production from non-transformed yeasts

According to the literature, wild-type (wt) yeast comprises a D-arabinono-1,4-lactone oxidase activity with a broad substrate specificity (Huh W.K. et al., 1994, Eur. J. Biochem. 225, 1073-1079). Such activity has been demonstrated *in vitro.* To determine whether the substrates or the product could cross the cell membrane, we incubated three different yeast strains (*S*. *cerevisiae* GRF18U and W3031B, as well as *Z. bailii* ATCC 60483) with L-galactono-1,4-lactone (the last precursor of the plant biosynthetic pathway leading to ascorbic acid) or L-gulono-1,4-lactone (the last precursor of the animal metabolic pathway). As shown in Figure 3, both of the substances can be internalized into the yeast cell and can be converted to ascorbic acid. No ascorbic acid was accumulated in the culture broth (not shown) but significant amounts were measured in whole cell extracts.

The next prior precursor in the plant pathway is L-galactose. Figure 4 shows the results of incubations of yeast cells with this substrate. *S. cerevisiae, Z. bailii,* and *K. lactis* are able to produce ascorbic acid from this compound, and in this case ascorbic acid is accumulated inside of the cell in significant amount (Fig. 4).

### 3. Ascorbic acid production and accumulation in the medium from transformed yeasts

We cloned the genes coding for D-arabinono-1,4-lactone oxidase (ALO), D-arabinose dehydrogenase (ARA), L-galactono-1,4-lactone dehydrogenase (AGD), L-galactose dehydrogenase (LGDH), L-gulono-1,4-lactone oxidase (RGLO) and aldonolactonase (AL). These genes were cloned into available yeast expression vectors as outlined in materials and methods. In short, the TPI promoter, a naturally strong and constitutive promoter of *S. cerevisiae,* drives the expression of the genes in question. Upon incubation of *S*. *cerevisiae* GRF18U transformed with AGD or ALO with L-galactono-1,4-lactone, the cells not only accumulated ascorbic acid intracellularly (not shown), but also, surprisingly, accumulated considerable amounts of ascorbic acid into the culture broth (Figure 5). This was also true for the same transformed cells incubated with L-galactose (Figure 6). Cotransformation of L-galactose dehydrogenase or D-arabinose dehydrogenase significantly increased the ability of the respective yeast strain to convert L-galactose to ascorbic acid (Figure 6). Results similar to those described above for recombinant GRF18U expressing (a) ALO; (b) LGDH and ALO; or (c) ARA and ALO, cultured in media comprising L-galactose, were seen with recombinant *S. cerevisiae* W3031B expressing the corresponding enzymes and cultured in media comprising L-galactose. (Results not shown.) Figure 7 shows data of a high-density culture converting L-galactose to ascorbic acid. The respective yeast strains were grown overnight in standard minimal medium. The next day, the cells were aseptically centrifuged and the pellet was resuspended in 1/10 of the supernatant to concentrate the cells 10 times. Then, 250 mg l⁻¹ of L-galactose were added and the cultures were incubated under standard conditions for 6 days. After 6 days the strain transformed with ALO and LGDH accumulated over 70 mg ascorbic acid per liter culture medium. 30 mg 1⁻¹ ascorbic acid were accumulated intracellularly (not shown). Taking these two values together corresponds to a conversion of around 40% of the L-galactose added.

In order to test the ability to produce ascorbic acid from a different yeast, *Z. bailii* ATCC 36947 was transformed with ALO and/or LGDH. The transformed cells were incubated with L-galactose under the same conditions as described for the transformed *S*. *cerevisiae* (Figure 8). Just as with the examples concerning *S. cerevisiae,* described above, accumulation of ascorbate was observed in the culture broth. The wild-type *Z. bailii* cells were able to accumulate a minor amount of ascorbic acid upon incubation with L-galactose. Transformation of the cells with a plasmid conferring resistance to the antibiotic G418 or hygromycin and incubation of the cells with the respective antibiotic appear to have abolished this endogenous ability. Functional expression of ALO, or ALO and LGDH results in a marked accumulation of ascorbic acid in the culture broth. The lower accumulation of ascorbate by cells expressing ALO incubated with both antibiotics as compared to the cells expressing ALO incubated with only G418, indicates that either the presence of hygromycin or the expression of the hygromycin resistance gene significantly interferes with the ascorbic acid accumulation/or detection. The higher accumulation of ascorbate in cultures of cells expressing ALO and LGDH compared to ascorbate levels in cultures of cells expressing only ALO, when the cells in both cases are cultured in the presence of both G418 and hygromycin indicates that LGDH is functionally expressed in *Z. bailii.*

Upon incubation of *S. cerevisiae* GRF18U transformed with RGLO in the presence of L-gulono-1,4-lactone, the cells accumulated ascorbic acid intracellularly (Figure 9), but not in the culture broth (data not shown). Untransformed cells converted a certain amount of L-gulono-1,4-lactone due to the crossreactivity of the endogenous ALO, as discussed above. Upon expression of RGLO in the cells the amount of ascorbic acid accumulated was significantly increased. Additional expression of AL resulted in lower levels of ascorbic acid accumulation. Due to hydrolysis, L-gulono-1,4-lactone is in equilibrium with L-gulonic acid when it is in an aqueous environment. This equilibrium strongly favors the free acid, but equilibrium is reached very slowly (days). Cells incubated with L-gulono-1,4-lactone are able to internalize the compound and to convert it to ascorbic acid. Upon functional co-expression of AL and both of the enzymes RGLO and ALO, all three enzymes are competing for substrate from the same equilibrium reaction between L-gulono-1,4-lactone and L-gulonic acid. The equilibrium with L-gulonic acid, which is not a substrate for ALO or for RGLO, is reached much more quickly, so the amount of the lactone that is converted by ALO/RGLO to ascorbic acid is reduced. The reverse reaction (lactonization of L-gulonic acid to L-gulono-1,4-lactone) hardly takes place in an aqueous environment without catalyzation by an enzyme, if at all. Therefore *S*. *cerevisiae* GRF18U that does not overexpress a protein or GRF18U expressing RGLO incubated with L-gulonic acid did not accumulate any ascorbic acid, either intracellularly (Figure 9) or extracellularly (data not shown). Upon expression of AL in *S. cerevisiae* GRF18U a small amount of L-gulonic acid was converted to L-gulono-1,4-lactone, which served as a substrate for ALO or ALO and RGLO. Therefore these cells were able to convert L-gulonic acid into ascorbic acid. This rendered the AL enzyme in addition to ALO and/or RGLO useful for the production of ascorbic acid from yeasts.

Upon incubation of *Z*. *bailii* ATCC36947 or ATCC60483 transformed with RGLO with L-gulono-1,4-lactone, the cells accumulated ascorbic acid intracellularly (Figure 10), but not in the culture broth (data not shown). Therefore, data obtained from these *Z. bailii* yeast strains were comparable to what observed from the yeast *S. cerevisiae.*

The following table summarizes the main examples reported in this invention. Note that "Not determ." in the table below is meant to indicate "not determined."

| Examples of Yeast | Examples of Gene overexpressed | Examples of Converted precursors | Production of Ascorbic acid | |
|---|---|---|---|---|
| | | | intracellular | extracellular |
| *S. cerevisiae* | No | L-galactono-1,4-lactone | yes | no |
| | | L-gulono-1,4-lactone | yes | no |
| | | L-galactose | yes | no |
| | | L-gulonic acid | no | no |
| *K. lactis* | No | L-galactose | yes | no |
| *Z. bailii* | No | L-galactono-1,4-lactone | yes | no |
| | | L-gulono-1,4-lactone | no | no |
| | | L-galactose | yes | yes |
| *S. cerevisiae* | AGD | L-galactono-1,4-lactone | yes | yes |
| *S. cerevisiae* | LGDH | L-galactose | yes | no |
| *S. cerevisiae* | LGDH +ALO or AGD | L-galactose | yes | yes |
| *S. cerevisiae* | ARA +ALO | L-galactose | yes | yes |
| *S. cerevisiae* | RGLO | L-gulono -1,4-lactone L-gulonic acid | yes | no |
| *S. cerevisiae* | AL | L-gulono -1,4-lactone L-gulonic acid | yes | no |
| *S. cerevisiae* | RGLO + AL | L-gulono -1,4-lactone L-gulonic acid | yes | no |
| *Z. bailii* | RGLO | L-gulono -1,4-lactone | yes | no |
| *Z. bailii* | LGDH | L-galactose | Not determ. | no |
| *Z. bailii* | ALO | L-galactose | Not determ. | yes |
| *Z. bailii* | LGDH + ALO | L-galactose | Not determ. | yes |

While the compositions and methods and yeast strains of this invention have been described in terms of preferred embodiments, it will be apparent to those of skill in the art that variations may be applied without departing from the concept, spirit and scope of the invention.

### REFERENCES

The following references, to the extent that they provide exemplary procedural or other details supplementary to those set forth herein, are specifically incorporated herein by reference.
[1] Agatep, R., R.D. Kirkpatrick, D.L. Parchaliuk, R.A. Woods, and R.D. Gietz, 1998, Transformation of Saccharomyces cerevisiae by the lithium acetate/single-stranded carrier DNA/polyethylene glycol (LiAc/ss-DNA/PEG) protocol. Technical Tips Online (http://tto.trends.com).
[2] Axelsen, Bock, and Krøll, In N. H. Axelsen, J. Krøll, and B. Weeks, editors, A Manual of Quantitative Immunoelectrophoresis, Blackwell Scientific Publications, 1973, Chapter 10.
[3] Berry, A., Running, J., Severson, D.K. and Burlingame, R.P., 1999, Vitamin C Production in Microorganisms and Plants, International Patent Application, WO99/64618.
[4] Bleeg, H.S. and Christensen, F., 1982, Biosynthesis of Ascorbate in Yeast, Purification of L-Galactono-1,4-lactone Oxidase with Properties Different from Mammalian L-Gulonolactone Oxidase, Eur. J. Biochem. 127,391-96.
[5] Bock and Axelsen, In N. H. Axelsen, J. Krøll, and B. Weeks, editors, A Manual of Quantitative Immunoelectrophoresis, Blackwell Scientific Publications, 1973, Chapter 11.
[6] Daruwala, R., Song, J., Koh, W.S., Rumsey, S.C. and Levine, M., 1999, Cloning and functional characterization of the human sodium-dependent vitamin C transporters hSVCT1 and hSVCT2. FEBS Lett. 460, 480-484.
[7] Dumbrava, VA. and Pall, M.L., 1987, Control of nucleotide and erythroascorbic acid pools by cyclic AMP in Neurospora crassa. Biochim Biophys Acta. 926, 331-338.
[8] Gietz, R.D. and Sugino, A., 1988, New yeast-Escherichia coli shuttle vectors constructed with in vitro mutagenized genes lacking six-base pair restriction sites. Gene 74, 527-534.
[9] Goldstein, A.L. and McCusker, J.H., 1999, Three New Dominant Drug Resistance Cassettes for Gene Disruption in Saccharomyces cerevisiae. Yeast 15, 1541-1553.
[10] Hancock, R.D., Galpin, J.R., and Viola, R., 2000, Biosynthesis of L-ascorbic acid (vitamin C) by Saccharomyces cerevisiae. FEMS Microbiol. Lett. 186, 245-250.
[11] Harboe and Ingild, In N. H. Axelsen, J. Krøll, and B. Weeks, editors, A Manual of Quantitative Immunoelectrophoresis, Blackwell Scientific Publications, 1973, Chapter 23.
[12] Harlow, E. and Lane, D., editors, 1988, Antibodies, A Laboratory Manual, Cold Spring Harbor Press, Cold Spring Harbor, New York.
[13] Huh, W.K., Lee, B.H., Kim, S.T., Kim, Y.R., Rhie, G.E., Baek, Y.W., Hwang, C.S., Lee, S.J. and Kang, S.O., 1998, D-Erythroascorbic acid is an important antioxidant molecule in S. cerevisiae, Mol. Microb. 30, 895-903.
[14] Huh, W.K., Kim, S.T., Yang, K.S., Seok, Y.J., Hah, Y.C. and Kang, S.O., 1994, Characterisation of D-arabinono-1,4-lactone oxidase from Candida albicans ATCC 10231, Eur. J. Biochem. 225, 1073-1079.
[15] Johnstone and Thorpe, Immunochemistry in Practice, Blackwell Scientific Publications, 1982, 27-31.
[16] Kim, S.T., Huh, W.K., Kim, J.Y., Hwang, S.W. and Kang, S.O., 1996, D-Arabinose dehydrogenase and biosynthesis of erythroascorbic acid in Candida albicans, BBA 1297, 1-8.
[17] Kim, S.T., Huh, W.K., Lee, B.H. and Kang, S.O., 1998, D-Arabinose dehydrogenase and its gene from Saccharomyces cerevisiae, BBA 1429, 29-39.
[18] Koshizaka T., Nishikimi M., Ozawa T. and Yagi K., 1988, Isolation and sequence analysis of a complementary DNA encoding rat liver L-gulono-gamma-lactone oxidase, a key enzyme for L-ascorbic acid biosynthesis. J. Biol. Chem. 263, 1619-1621.
[19] Kreger-van Rij, N. J. W., 1987, The Yeasts, Vol. 1 of Biology of Yeasts, Ch. 2, A. H. Rose and J. S. Harrison, Eds. Academic Press, London.
[20] Kumar, M. 2000 Production of ascorbic acid using yeast, International patent application, WO 00/34502.
[21] Lee, B.H., Huh, W.K., Kim, S.T., Lee, J.S. and Kang, S.O., 1999, Bacterial Production of D-Erythroascorbic Acid and L-Ascorbic Acid through Functional Expression of Saccharomyces cerevisiae D-Arabinono-1,4-Lactone Oxidase in Escherichia coli, App. Env. Microb. 65, 4685-4687.
[22] Loewus M.W. et al., 1990, Plant. Physiol. 94,1492-1495.
[23] Lowry, O.H., Rosebrough, N.J., Farr, A.L. and Randall, R.J., 1951, Protein Measurement with the Folin Phenol Reagent, J.Biol.Chem. 193, 265-275.
[24] Minet, M., Dufour, M.E. and Lacroute, F., 1992, Complementation of Saccharomyces cerevisiae auxotrophic mutants by Arabidopsis thaliana cDNAs. Plant J. 2, 417-422.
[25] Nishikimi, M., Noguchi, E. and Yagi, K., 1978, Occurrence in Yeast of L-Galactonolactone Oxidase Which is Similar to a Key Enzyme for Ascorbic Acid Biosynthesis in Animals, L-Gulonolactone Oxidase, Arch. Biochem. Biophys. 191, 479-486.
[26] Østergaard, J., Persiau, G., Davey, M.W., Bauw, G. and Van Montagu, M., 1997, Isolation of a cDNA Coding for L-Galactono-Lactone Dehydrogenase, an Enzyme involved in the Biosynthesis of Ascorbic Acid in Plants, J. Biol. Chem. 272, 30009-30016.
[27] Padh, H., 1990, Cellular functions of ascorbic acid. Biochem Cell Biol. 68, 1166-1173.
[28] Reichstein, T., Process for the Manufacture of Levoascorbic Acid (Vitamin C), United States Pat. No. 2,265,121.
[29] Roland, J.F., Cayle, T., Dinwoodie, R.C. and Mehnert, D.W., 1986, Fermentation Production of Ascorbic Acid from L-Galactonic Substrate, United States Patent 4,595,659.
[30] Roland, J.F., Cayle, T., Dinwoodie, R.C. and Mehnert, D.W., 1990, Bioconversion Production of Ascorbic Acid with L-Galactono-1,4-Oxidase, United States Patent 4,916,068.
[31] Roland, J.F., Cayle, T., Dinwoodie, R.C. and Mehnert, D.W., 1986, Fermentation production of ascorbic acid from L-galactonic substrate, International Patent Application, WO85/01745.
[32] Spickett, C.M., Smirnoff, N. and Pitt, A.R. 2000, The biosynthesis of erythroascorbate in Saccharomyces cerevisiae and its role as an antioxidant. Free Radic. Biol. Med. 28,183-192.
[33] Sambrook, J., Fritsch, E. F., and Maniatus T., 1989, Molecular Cloning, A Laboratory Manual, 2nd edition, Cold Spring Harbor, New York.
[34] Smirnoff, N. and Wheeler, G., 1999, Plant Galactose Dehydrogenase, International Patent Application, WO99/33995.
[35] Sullivan, M.X. and Clarke, H.C.N., 1955, A highly specific procedure for ascorbic acid, Assoc. Off. Agr. Chem. 38, 514-518.
[36] Wach, A., Brachat, A., Pohlmann, R., and Philippsen, P., 1994, New heterologous modules for classical or PCR-based gene disruptions in Saccharomyces cerevisiae. Yeast 10, 1793-1808.
[37] Wésolowski-Louvel, M., Prior, C., Bornecque, D. and Fukuhara, H., 1992, Rag- mutations involved in glucose metabolism in yeast: isolation and genetic characterization. Yeast 8, 711-719.
[38] Wheeler, G.L., Jones, M.A. and Smirnoff, N., 1998, The biosynthetic pathway of vitamin C in higher plants, Nature 393, 365-369.

## Claims

1. A method of generating ascorbic acid or a salt thereof, comprising:
a) obtaining a recombinant *Saccharomyces cerevisiae* or *Zygosaccharomyces bailii* yeast functionally transformed with a coding region encoding a first enzyme D-arabinose dehydrogenase (ARA), wherein the yeast is capable of converting an ascorbic acid precursor into ascorbic acid or a salt thereof,
b) culturing the recombinant yeast in a medium comprising an ascorbic acid precursor, thereby forming ascorbic acid or a salt thereof, wherein the yeast accumulates ascorbic acid in the medium at levels greater than background, and
c) isolating the ascorbic acid or the salt thereof.

2. The method of claim 1, wherein the yeast is selected from *S. cerevisiae* strains NRRL Y-30326 and NRRL Y-30329, wherein NRRL refers to the Northern Regional Research Laboratory, Agricultural Research Service, United States Department of Agriculture.

3. The method of claim 1, wherein the ARA enzyme has at least 70% identity with SEQ ID NO: 20 or at least 70% identity with nucleotides 285 through 1319 of SEQ ID NO: 21.

4. The method of claim 1, wherein the ARA enzyme comprises the amino acid sequences GXRXXDXAXXXXXEXXXG (SEQ ID NO: 13) and GXXN (SEQ ID NO: 26).

5. The method of claim 1, wherein the yeast is further functionally transformed with a coding region encoding a second enzyme selected from D-arabinono-1,4-lactone oxidase (ALO) and L-galactono-1,4-lactone dehydrogenase (AGD).

6. The method of claim 5, wherein the ALO enzyme has at least 70% identity with SEQ ID NO: 5 or SEQ ID NO: 7 or at least 70% identity with SEQ ID NO: 6 or nucleotides 4 through 1584 of SEQ ID NO: 8.

7. The method of claim 5, wherein the AGD enzyme has at least 70% identity with SEQ ID NO: 1 or SEQ ID NO: 3 or at least 70% identity with SEQ ID NO: 2 or nucleotides 56 through 1858 of SEQ ID NO: 4.

8. The method of claim 5, wherein the coding region encoding the second enzyme is linked to a promoter active in the yeast.

9. The method of claim 8, wherein the promoter is the *S. cerevisiae* triosephosphateisomerase (TPI) promoter.

10. The method of claim 1, wherein the coding region encoding the ARA enzyme was isolated from *S. cerevisiae.*

11. The method of claim 5, wherein the coding region encoding the ALO enzyme was isolated from *S*. *cerevisiae.*

12. The method of claim 5, wherein the coding region encoding the AGD enzyme was isolated from *A. thaliana.*

13. The method of claim 1, wherein the coding region encoding the first enzyme encodes an ARA enzyme that hybridizes to a complementary strand of the polynucleotide set forth in nucleotides 285 through 1319 of SEQ ID NO: 21; and wherein the coding region hybridizes to the complementary strand of the polynucleotide under stringent hybridization conditions.

14. The method of claim 1, wherein the coding region encoding the first enzyme encodes an ARA enzyme having the same immunological properties of an enzyme having SEQ ID NO: 20.

15. The method of claim 5, wherein the coding region encoding the second enzyme encodes an ALO enzyme that hybridizes to a complementary strand of the polynucleotide set forth in SEQ ID NO:6; and wherein the coding region hybridizes to the complementary strand of the polynucleotide under stringent hybridization conditions.

16. The method of claim 5, wherein the coding region encoding the second enzyme encodes an ALO enzyme having the same immunological properties of an enzyme having SEQ ID NO: 5 or SEQ ID NO: 7.

17. The method of claim 5, wherein the coding region encoding the second enzyme encodes an AGD enzyme that hybridizes to a complementary strand of the polynucleotide set forth in SEQ ID NO: 2; and wherein the coding region hybridizes to the complementary strand of the polynucleotide under stringent hybridization conditions.

18. The method of claim 5, wherein the coding region encoding the second enzyme encodes an AGD enzyme having the same immunological properties of an enzyme having SEQ ID NO: 1 or SEQ ID NO: 3.

19. The method of claim 1, wherein the ascorbic acid precursor is selected from the group consisting of monosaccharides, oligosaccharides, polysaccharides, C₁-C₆ alcohols, C₂-C₆ polyols, organic acids, and C₅-C₆ lactones.

20. The method of claim 1, wherein the ascorbic acid precursor is selected from the group consisting of trehalose; raffinose; ethanol; xylose; xylulose; maltose; glycerol; fructose; arabinose; D-glucose-6-P; D-glucose-1-P; UDP-D-glucose; UDP-glucuronic acid; D-glucuronic acid-1-P; D-glucuronic acid; D-glucurono lactone; L-gulonic acid; D-fructose-6-P; D-mannose-6-P; D-mannose-1-P; GDP-D-mannose; GDP-L-galactose; L-galactose-1-P; lactose; sucrose; starch; cellulose; citrate; methanol; formaldehyde; formate; methylamine; alanine; oleic acid; L-galactono-1,4-lactone; D-glucose; L-gulono-1,4-lactone; L-galactose; and L-gulonic acid.

21. The method of claim 1, wherein the ascorbic acid precursor is selected from L-galactono-1,4-lactone; D-glucose; L-gulono-1,4-lactone; L-galactose; and L-gulonic acid.

22. The method of claim 1, wherein the ascorbic acid precursor is also a carbon source for the recombinant yeast.

23. The method of claim 1, wherein the isolating step comprises lysing the yeast.

24. The method of claim 23, wherein the isolating step further comprises centrifugation, filtration, microfiltration, ultrafiltration, nanofiltration, liquid-liquid extraction, activated carbon, crystallization, enzymatic treatment with nuclease or protease, or chromatography.

25. The method of claim 1, wherein the culturing step comprises accumulating ascorbic acid in the medium at a final concentration of at least 20 mg/l.

26. The method of claim 1, wherein the culturing step comprises accumulating ascorbic acid in the medium at a final concentration of at least 40 mg/l.

27. The method of claim 1, wherein the culturing step comprises accumulating ascorbic acid in the medium at a final concentration of at least 70 mg/l.

28. The method of claim 1, wherein the recombinant yeast is capable of converting at least 25% of the precursor to ascorbic acid.

29. The method of claim 1, wherein the recombinant yeast is capable of converting at least 35% of the precursor to ascorbic acid.

30. The method of claim 1, wherein the recombinant yeast is capable of converting at least 40% of the precursor to ascorbic acid.

31. A method of stabilizing ascorbic acid or a salt thereof in a medium, comprising: culturing a recombinant *Saccharomyces cerevisiae* or *Zygosaccharomyces bailii* yeast functionally transformed with a coding region encoding a first enzyme D-arabinose dehydrogenase (ARA) in a medium comprising ascorbic acid or a salt thereof, wherein ascorbic acid in the medium is produced by the yeast, the yeast produces ascorbic acid by converting at least one ascorbic acid precursor to ascorbic acid, and the medium comprises the ascorbic acid precursor.

32. The method of claim 31, wherein ascorbic acid or the salt thereof is added to the medium.

33. The method of claim 31, wherein the yeast is further functionally transformed with a coding region encoding a second enzyme selected from D-arabinono-1,4-lactone oxidase (ALO) and L-galactono-1,4-lactone dehydrogenase (AGD).

34. A *S. cerevisiae,* wherein the *S. cerevisiae* is functionally transformed with a coding region encoding D-arabinose dehydrogenase (ARA).

35. A *S. cerevisiae,* wherein the *S. cerevisiae* is functionally transformed with a coding region encoding D-arabinose dehydrogenase (ARA), and a coding region encoding D-arabinono-1,4-lactone oxidase (ALO) or L-galactono-1,4-lactone dehydrogenase (AGD).
